# EUROPEAN PATENT SPECIFICATION

(11) **EP 1 999 271 B1**
(45) Date of publication and mention of the grant of the patent: **03.07.2013**
(21) Application number: 05819260.0
(22) Date of filing: 22.12.2005
(51) Int. Cl.: C12Q 1/68

(54) **A METHOD OF IDENTIFYING INDUCED VARIABILITY IN IN VITRO CULTURES**
VERFAHREN ZUR IDENTIFIZIERUNG INDUZIERTER VARIABILITÄT IN IN-VITRO-KULTUREN
PROCÉDÉ D'IDENTIFICATION DE LA VARIABILITÉ INDUITE DANS DES CULTURES IN VITRO

(43) Date of publication of application: 10.12.2008
(73) Proprietor: Instytut Hodowli I Aklimatyzacji Roslin (Plant Breeding and Acclimatization Institute), 05-870 Radzikow (PL)
(72) Inventor: BEDNAREK, Piotr, PL-01-960 Warszawa (PL); ZIMNY, Janusz, PL-05-870 Blonie (PL)
(74) Representative: Twardowska, Aleksandra
(86) International application number: PCT/PL2005/000083
(87) International publication number: WO 2007/073221

(56) References cited:
- WO-A-99/53100
- OLESZCZUK S ET AL: "THE APPLICATION OF THE AFLP METHOD TO DETERMINE THE PURITY OF HOMOZYGOUS LINES OF BARLEY (HOREDUM VULGARE L.)" CELLULAR AND MOLECULAR BIOLOGY LETTERS, UNIVERSITY OF WROCAW. INSTITUTE OF BIOCHEMISTRY, WROCAW, PL, vol. 7, no. 2B, 2002, page 777783, XP001246891 ISSN: 1425-8153
- PERAZA-ECHEVERRIA SANTY ET AL: "Detection of DNA methylation changes in micropropagated banana plants using methylation-sensitive amplification polymorphism (MSAP)" PLANT SCIENCE (SHANNON), vol. 161, no. 2, July 2001 (2001-07), pages 359-367, XP002395612 ISSN: 0168-9452
- LINACERO R ET AL: "Hot spots of DNA instability revealed through the study of somaclonal variation in rye" THEORETICAL AND APPLIED GENETICS, vol. 100, no. 3-4, February 2000 (2000-02), pages 506-511, XP002395613 ISSN: 0040-5752

## Description

The subject of the present invention is use of the AFLP technique which is composed of the stages: isolation of genomic DNA, digestion of the genomic DNA with one pair of restriction endonucleases and digestion of another portion of the same DNA with another pair of restriction endonucleases, annealing of synthetic heteroduplexes to the free restriction ends, initial amplification of fragments obtained in such a manner by way of primary PCR, selective amplification, separation of the PCR products in a polyacrylamide gel, visual profile analysis for the method for the identification of variability induced by the derivation method in *in vitro* cultures

It is a well established phenomenon that, following plant regeneration from non-differentiated tissue, the resulting population of clonal individuals is not phenotypically or genetically homogeneous. Both somaclonal and gametoclonal events contribute to this tissue culture induced variation. The term somaclonal variation was introduced by Larkin and Scowcroft [Larkin and Scowcroft 1981] to describe variation arising during somatic cell culture, while gametoclonal variation represents heritable variation arising during the culture of cells of gametic origin. The genetic basis underlying somaclonal variation remains controversial [Bouman and De Klerk 2001, Bregitzer, et al. 1998, Fourré, et al. 1997, Hossain, et al. 2003, Linacero, et al. 2000, Munthali, et al. 1996] and both the mechanisms by which it is induced, and the ways in which can be detected and exploited have been widely discussed in the literature [Brar and Jain 1998, Remotti 1998]. Genetic (DNA point mutagenesis, microsatellite sequence amplification [Linacero, et al. 2000] and transposon movement (Peshke and Phillips 1991] and epigenetic (localized changes in DNA methylation state (Jaligot, et al. 2002, Jaligot, et al. 2000))) events provide a molecular route to its induction. It is generally accepted that *in vitro* systems that require prolonged passage through a callus or cell suspension phase are the most vulnerable to mutation (Arene, et al. 1993, Freyssinet and Freyssinet 1988, Roseland, et al. 1991, Thomas, et al. 1982), and it has therefore been suggested that the most effective means of minimizing the variation is to attempt to induce somatic embryogenesis as rapidly as possible (Gray, et al. 1995). The definition of the molecular changes underlying *de novo* somaclonal variation and the quantification of its frequency have been hindered by methodological difficulties (Gaj 2001), and this is reflected in the limited description of this variation (mostly at the phenotypic level) in the literature (Guzy-Wróblewska and Szarejko 2003). However, a lack of visually detectable changes cannot be taken to infer a lack of variation at the molecular level (Gaj 2001).

DNA fingerprinting technology has the potential to detect DNA changes with growing precision and efficiency. However, neither RFLP- nor RAPD-based approaches have been particularly successful in recognizing somaclonal variants (Devaux, et al. 1993). Munthali et al. (Munthali, et al. 1996) identified only three variants out of 5607 RAPD products among a set of regenerated beet plants, and a similarly low frequency was observed in *Begonia* (Bouman and De Klerk 2001). Somewhat better results have been achieved in studies of protoplast-derived regenerants of *Lolium* (Wang, et al. 1993), and from long-term wheat cell culture suspensions (Brown, et al. 1993). The AFLP technique is potentially more appropriate as a detection platform, since it both identifies a relatively large number of amplified fragments, and is more experimentally robust than RAPD, as a result of its use of a more stringent PCR regime. Despite this, relatively few reports of its use to detect variation in methylation pattern or somaclonal variation have been published to date (Cervera, et al. 2002, Portis, et al. 2003, Vendrame, et al. 1999). As an example, it was able to unequivocally discriminate between embryogenic culture lines of *Carya illinoinensis* and was able to group embryos originating from a given line (Vendrame, et al. 1999).

A feature of AFLP is the flexibility of choice for the two restriction enzymes needed for the initial digestion of template DNA. Where one or both are sensitive to methylation, the loss or gain of fragments in a profile can be due either to gain/loss of restriction sites (sequence variants) or to differential methylation of particular recognition sites, as pointed out by Donini et al. (Donini, et al. 1997). Isoschizomeric comparisons, in which one treatment uses a methylation-sensitive, and the other a methylation-insensitive enzyme have been explored in *Arabidopsis* (Cervera, et al. 2002). This method, termed methylation sensitive amplified polymorphism (MSAP), is particularly suited to the analysis of the molecular basis of somaclonal variation. The present study describes the elaboration and application of an analytical method, which enables the detection of tissue culture-induced variation in barley *(Hordeum vulgare L.)* both at the nucleotide sequence and at the DNA methylation level. Our aim was further to compare the frequency and patterns of variation between two different sources of explants (immature embryos and microspores) and to evaluate the potential of the approach to quantify the contributions of methylation and sequence alteration to overall variation. We propose a method flexible enough to study different parts of the genome, and tailored to giving either a qualitative and quantitative description of tissue culture-induced variation.

Patent description US6300071 (published 2001-10-09) describes a method for detecting nucleic acid methylation using AFLP(TM). The invention relates to a method for analyzing of determining the methylation pattern of a starting DNA and/or for distinguishing between methylated and non-methylated sites in the starting DNA, comprising at least(A) generating a first DNA fingerprint, containing bands corresponding to both the methylated and non-methylated sites of interest; and/or(B) generating a second DNA fingerprint, containing bands corresponding only to the methylated sites of interest; and optionally comprising(C) generating a third DNA fingerprint, containing bands corresponding only to the non-methylated sites of interest; and optionally further comprising(D) analysing the fingerprint(s) thus obtained. The fingerprints are preferably generated using AFLP, by means of a frequent cutter and a methylation sensitive rare cutter. The invention further relates to specific methods for generating the above first and second DNA fingerprint by means of AFLP, and kits for use with said methods.

Patent description WO9953100 (published 1999-10-21) describes a method for finding genetic markers of somaclonal variation. The invention provides a method for obtaining molecular markers for use as a diagnostic and quality control tool to identify genomic polymorphisms that arise during the process of tissue culture of in vitro propagated plants. By using a representational difference analysis (RDA) adapted for plant genomes, a set of nucleic acid difference sequences between normal and off-type plant genomes are obtained. The invention further provides a method for isolating sets of variant sequences which are common to many naturally occurring or tissue culture-generated off-types of the same cultivar or species, in addition to variant sequences present in all off-types, regardless of the phenotypic mutation, and/or in all off-types that exhibit the same mutation. Detection of somaclonal variation by the method of the invention may present an opportunity to optimize tissue culture conditions and to optimize plant multiplication rates without producing a significant number of off-types.

Despite the above mentioned research on methods of identifying induced variability in *in vitro* cultures applicable in such uses as obtaining genetically equivalent lines, and which also facilitate the performance of broadly understood selection of plant material derived via *in vitro* cultures for cultivation purposes, as well as the possibility of producing a molecular tool for the identification of epigenetic characteristics for cultivation purposes, a need still exists to produce an effective solution for the quantitative and qualitative evaluation of variance induced in *in vitro* cultures and somaclonal variance.

The goal of the present invention is to provide tools which could be used into obtain a method of quantitative and qualitative estimation of induced variability in *in vitro* cultures as well as stably inherited changes in subsequent generative generations. The goal of the present solution is to produce genetically stable lines, including doubled haploids, to determine the level of their genetic-epigenetic variability, to facilitate the selection of plant material derived via *in vitro* culturing for cultivation purposes, as well as producing easily adaptable molecular tools for the identification of their epigenetic characteristics for culturing purposes. The goal is to obtain a method of quantitatively characterizing the variability induced in *in vitro* cultures as well as variation inherited subsequent generative generations, which would facilitate the evaluation of individual types of variability (sequence and methylation variability, divided into more specific subtypes) which would allow one to determine which of the types (subtypes) of variability is dominant, whether sequence variability is caused by mobile element migration, changes in micro-and macrosatellite regions, within gene families, etc.

The realization of such a stated goal to provide solutions to the problems described in the state of the art, in order to provide tools which could be used into obtain a method of quantitative and qualitative estimation of induced variability in *in vitro* cultures as well as variation inherited as a result of generative propagation, which would facilitate the selection of plant materials derived *through in vitro* culture for cultivation purposes, the application of said methodology to determine what percentage of variability sequence or methylation variability (methylated and non-methylated sites) was stably passed onto plant progeny, were achieved in the present invention.

The topic of the subject invention is use of the AFLP technique which is composed of the stages: isolation of genomic DNA, digestion of the genomic DNA with one pair of restriction endonucleases and digestion of another portion of the same DNA with another pair of restriction endonucleases, annealing of synthetic heteroduplexes to the free restriction ends, initial amplification of fragments obtained in such a manner by way of primary PCR, selective amplification, separation of the PCR products in a polyacrylamide gel, visual profile analysis for the method for the identification of variability induced by the derivation method in *in vitro* cultures at the level of regenerant plants, as well as variation inherited as a result of generative propagation consisting of the following stages:
1) estimation of the repeatability of the two AFLP technique variants: repeatability control of AFLP based on the identity of several DNA samples of the same plant through the use of AFLP and isoschizomers both sensitive and insensitive to methylation at the restriction-site and/or adjacent areas as well as a representative number of pairs of selective primers identical in all AFLP analyses;
2) estimation of the repeatability of the two AFLP technique variants: repeatability control of AFLP based on the identity of several DNA samples of the same plant through the use of AFLP and isoschizomers both sensitive and insensitive to methylation at the restriction-site and/or adjacent areas as well as a representative number of pairs of selective primers using a different set of selective AFLP primers than those used in the other AFLP analyses;
3) identification of variability induced by the method of derivation of the regenerants in relation to the donor plant is performed in two AFLP variants using isoschizomers both sensitive and insensitive to methylation at the restriction-site and/or adjacent areas as well as a representative number of pairs of selective primers identical in all AFLP analyses;
4) the identification of the tissue culture induced variability among regenerants in relation to the donor plant is performed in two AFLP technique variants utilising isoschizomers both sensitive and insensitive to methylation at the restriction-site and/or adjacent areas as well as a representative number of pairs of selective primers identical in all AFLP analyses;
5) the identification of somaclonal variability among regenerant progeny in relation to the donor plant is carried out using two AFLP technique variants utilising isoschizomers both sensitive and insensitive to methylation at the restriction-site and/or adjacent areas as well as a representative number of pairs of selective primers identical in all AFLP analyses;
6) the molecular analysis of the experimental material using the AFLP technique taking into account isoschizomers both sensitive and insensitive to methylation at the restriction-site and/or adjacent areas as well as a representative number of pairs of selective primers identical in all AFLP analyses is comprised of the following stages:
   a) analyses of AFLP patterns of donor plants with respect to genetic and epigenetic variability induced during the generative propagation of an individual plant, a control of generative propagation,
   b) analyses of AFLP patterns for the control of the reproducibility of both AFLP technique variants,
   c) analysis of AFLP patterns for regenerants in comparison to donor plants or separate régénérants in relation to appropriate donor plants;
   d) danalyses of AFLP patterns for donor plant, its regenerant and regenerant progeny, or distinct regenerant progeny lines along with appropriate donor and its regenerant plants;
7) identification of the 14 types of events recognized at the binary matrix level for systems sensitive and insensitive to methylation, which are the result of a comparison between the donor plant with regenerants and between donor plant with regenerant progeny simultaneously in two AFLP systems, wherein the identification of event types is carried out individually for each regenerant and as bulk for regenerant progenies and is represented as 14 binary matrices which describe each of the events independently, subdivided into methods of derivation; wherein the columns represent consecutive regenerants or bulks and rows represent consecutive AFLP fragments;
8) assignment of certain event types to a given type of variability induced the method of derivation in relation to each regenerant;
9) identification of the 14 types of events recognized at the binary matrix level for systems sensitive and insensitive to methylation, which are the result of a comparison between the donor plant with regenerant simultaneously in two AFLP systems;
10) identification of the 14 types of events recognized at the binary matrix level for systems sensitive and insensitive to methylation, which are the result of a comparison between the donor plant with regenerant progeny simultaneously in two AFLP systems, where the identification of event types is performed through the summation of the genotypes of a given summary progeny, and through the comparison of such a total AFLP profile of regenerant progeny with the donor plant profile,
11) the bulk regenerant progeny genotype expressed via an AFLP profile for systems sensitive and insensitive to methylation at the level of individual AFLP fragments has a different value from that of the donor plant in a given AFLP system, so long as a given fragment within it is polymorphic, or is totally different from this value for the donor plant; in the opposite case the values for the donor plant and bulk regenerant progeny for a given AFLP fragment in a given AFLP system are identical,
12) production of a quantitative matrix for regenerants, where the columns contain data for consecutive regenerants grouped by the method of derivation, and the rows contain the 14 types of events which may be assigned to each regenerant progeny;
13) production of a quantitative matrix for regenerant progeny, where the columns contain bulk data for regenerant progeny grouped by the method of derivation, and the rows contain the 14 types of events which may be assigned to each bulk regenerant progeny;
14) production of a quantitative matrix for events in regenerants, where this is a quantitative matrix for post-transposition regenerants, where the columns contain 14 events and the rows contain regenerants grouped by the method of derivation;
15) calculation of the total number of events based on the binary matrix for individual methods of derivation of regenerants as well as for regenerants, where the total number of events is calculated as a product of the number of AFLP fragments identified in both AFLP systems for all pairs of selective primers multiplied by the number of regenerants derived by a given method;
16) calculation of the total number of events based on the binary matrix for individual methods of derivation of regenerants as well as for regenerant progenies in bulk, where the total number of events corresponds to the number of DNA fragments identical or different in both AFLP systems for all pairs of selective primers used in the analysis;
17) calculation of the error stemming from generative propagation is carried out based on the binary matrix of donor plants and a single plant; determination of the number of events different from those in a single plant belonging to the donor plants, and the calculation of the total number of events for the donor plants;
18) calculation of the error stemming from the AFLP technique is carried out based on the binary matrix for DNA samples obtained from the same plant and subjected to the stages of the AFLP technique using isoschizomers sensitive and insensitive to methylation at the restriction site or its immediate surroundings as well as a representative number of selective starter pairs identical in all AFLP systems, where the total number of events and the number of polymorphic events are calculated, and the latter number is expressed as a percentage;
19) determination, based on binary matrices for regenerants and the donor plant, of groups of events describing: (a) sequence variation connected with any changes in DNA nucleotide sequences based on AFLP data from the system insensitive to methylation and (b) epigenetic variability, the *de novo* variability of methylation patterns, and additionally mixed variation which is sequence and methylation variability based on data for both AFLP systems;
20) determination, based on binary matrices for bulk regenerant progeny and the donor plant, of event groups describing (a) sequence variation comiected with all DNA nucleotide changes based on AFLP data from the system insensitive to methylation and (b) epigenetic variability, the *de novo* variability of methylation patterns, and additionally mixed variation which is sequence and methylation variability, and (c) inheritance of methylation patterns as related to the presence and/or lack of methylation at the restriction site or its vicinity, based on data for both AFLP systems;
21) identification of events particularly subject to the activity of factors inducing variability in *in vitro* cultures;
22) sequence analysis of individual event types and/or broad sequence variability, where variability is identified using AFLP technique variants using primers directed against conservative sequences within mobile elements, repeating sequences or the sequences of conservative gene families;
23) statistical analysis of the donor plant and its regenerants as well as regenerant progeny, and possibly distinct regenerants and regenerant progenies along with appropriate donor plants, as well as AFLP reproducibility control and generative propagation control:
   a) statistical analysis of the results of AFLP using parametric analysis, Mantel's test and/or Cronbach's alpha coefficient as well as molecular variance analysis using the *Fₛ,*or *Phiₛ,* coefficients;
   b) determination of the error of the tissue culture induced and somaclonal variability estimates, based on the Mantel test correlation coefficient;
   c) determination of the quantitative parameters of variability induced by the method of derivation of regenerants and somaclonal variability for regenerants, individual regenerant progenies, talcing into account the method of derivation and giving their statistical significance level;
   d) statistic determination of differences between individual types of events induced in *in vitro* cultures for regenerants, as well as bulk regenerant progeny, grouped by method of derivation and supplemented by their statistical significance using quantitative matrices of events;
   e) statistical analysis of the comparison between variability induced by the method of derivation as well as somaclonal variation, determined through the comparison of the donor plant profile, and the bulk profile of regenerant progeny and their types: sequence variability and/or broad sequence variability, *de novo* methylation, demethylation, mixed variability, inheritance of: methylation patterns, non-methylated sites or conservative sites in regenerant progeny, grouped by method of derivation as well as a comparison by method of derivation of regenerant progeny, where quantitative matrices are used for events;
   a) statistical comparisons of regenerants, and the bulk regenerant progenies for different methods of derivation for a given regenerant progeny, where quantitative matrices of the progeny are used;
   b) statistical comparison of the results of the above mentioned points for individual regenerants and bulk regenerant progenies amongst one another;
   h) statistical or qualitative comparison of somaclonal variation between individual plants being the generative progeny of regenerants;
   i) collation of the existing results in the form of a compilation indicating quantitative characteristics of variability induced by the method of derivation determined at the regenerant level, as well as quantitative characteristics of inherited variability determined at the level of regenerant progeny.

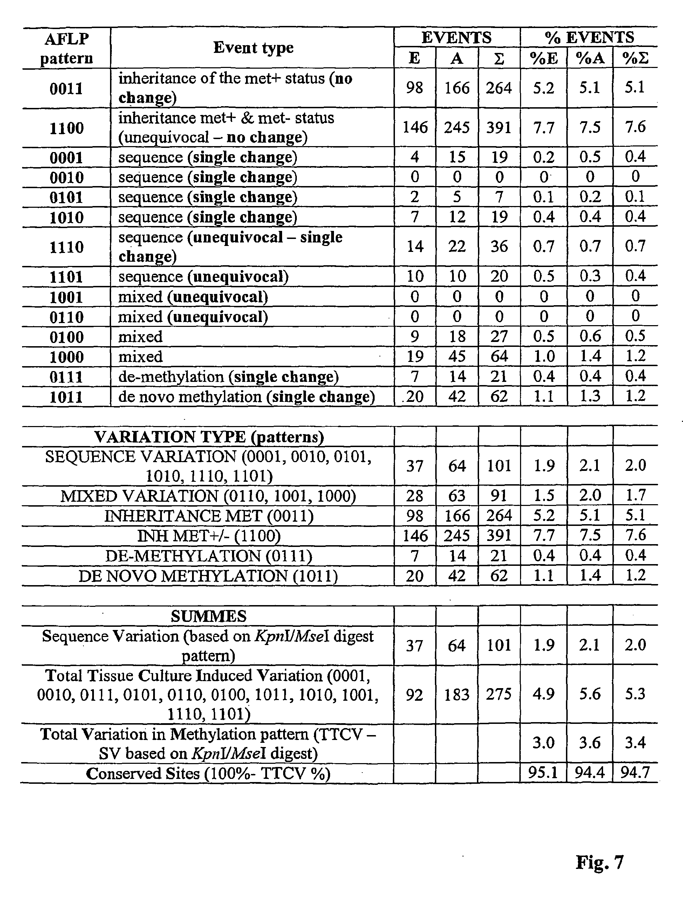

Preferentially the identification of the 14 types of events recognized at the level of binary matrix is selected from among the events represented in Fig. 7.

Preferentially to derive donor plants, a single plant is used which is a doubled haploid derived from an isolated microspore, wherein it is treated as a control of the genetic and epigenetic variability of donor plants.

Preferentially genetic and epigenetic variability of generatively propagated material, donor plants, is determined based on AFLP profiles obtained from restriction endonuclease isoschizomers recognizing and digesting identical sequences, but differing in their sensitivity to methylation at or about the restriction site, wherein one of the isoschizomers is insensitive to methylation.

Preferentially in a system sensitive and insensitive to methylation no differences are observed in the AFLP profiles of donor plants, then the level of variability induced through generative propagation and identified with a given pair of selective primers is insignificant.

Preferentially when there are differences in AFLP profiles for donor plants between a system sensitive and a system insensitive to methylation, a percentage portion of such an event is determined, along with the background level, generative propagation error.

Preferentially when there are differences in AFLP profiles for experimental replicants on the same DNA samples, the AFLP technique error is estimated in order to determine experimental error.

Preferentially the integral portion of the research material is composed of donor plants, along with a single plant, donor plant regenerants and separate regenerant progenies.

Preferentially the donor plant along with regenerants and regenerant progeny compose the core of research material for the study of variability induced by the method of derivation or somaclonal variability, respectively.

Preferentially the molecular analysis is performed using the AFLP technique in two variants, using isoschizomers differing in their sensitivity to methylation.

Preferentially one of the isoschizomers is completely insensitive to methylation.

Preferentially in the identification of segregated PCR products, and isotope marker is used which is introduced to the 5' end of the primer complementary to the adaptor annealed to the restriction site freed by the isoschizomers.

Preferentially other methods of visualising AFLP fragments are used, including fluorescence.

Preferentially the profiles of both AFLP variants for the donor plant along with its regenerants and taking into account the method of derivation and AFLP reproducibility control are performed on identical AFLP primer pairs.

Preferentially AFLP profile analysis and their conversion to binary matrices is based on the fact that the AFLP profiles of both restrictase systems for all pairs of selective primers are entered as a binary matrix into a spreadsheet, where the data set obtained for one pair of selective AFLP primers is placed below the previous one such that each column represents a total binary profile for a donor plant in one AFLP system, and the subsequent columns contain analogous data for regenerants and controls, wherein data for the second AFLP system is recorded in subsequent columns, wherein the bands from one system are ordered like those from the previous system (in rows), and where a given band does not appear a zero is entered in the appropriate row of the spreadsheet.

Preferentially AFLP profile analysis and their conversion to binary matrices is based on the fact that the AFLP profiles of both restrictase systems for all pairs of selective primers are entered as a binary matrix into a spreadsheet, where the data set obtained for one pair of selective AFLP primers is placed below the previous one such that each column represents a total binary profile for a donor plant in one AFLP system, and the subsequent columns contain analogous data for regenerant progenies and controls, wherein data for the second AFLP system is recorded in subsequent columns, wherein the bands from one system are ordered like those from the previous system (in rows), and where a given band does not appear a zero is entered in the appropriate row of the spreadsheet.

Preferentially AFLP profile for regenerant progeny is represented in the form of a bulk genotype per AFLP variant, sensitive and insensitive to methylation separately.

Preferentially the generative propagation control is carried out through the summation of the number of signals (events) from a single plant, where the number of changes is calculated as a percentage of the total number of events.

Preferentially the parametric analysis, Mantel's test correlation analysis and molecular variability analysis are performed using binary matrices of AFLP systems sensitive and insensitive to methylation for regenerants and/or regenerant progeny depending on whether the variability induced in culture or inherited by regenerant progeny are being analyzed.

Preferentially the factorial analysis is used as the graphical representation of the AFLP results.

Preferentially the identification of the 14 types of events recognized at the level of AFLP pattern and assigned to variability induced by the method of derivation is performed on the basis of AFLP patterns, wherein the donor plant profile is used along with regenerant profiles in both systems, and subsequently the profile of each regenerant is compared to the donor plant profile in order to identify these events.

Preferentially the identification of the 14 types of events recognized at the level of AFLP patterns and assigned to somaclonal variability is carried out on the basis of AFLP patterns, where the donor plant profile is used along with profiles in both AFLP systems, and the bulk profile of regenerant progeny is compared to the donor plant profile in order to identify event types, wherein it is assumed that in case of polymorphic AFLP fragments of the bulk regenerant progeny genotype, a value different than that of the donor plant is entered or a different value if it is different from that of the donor plant in all regenerant progenies for a given AFLP system.

Preferentially when the 14 event types comprise the basis for their segregation into groups: sequence variability, methylation variability (methylation and demethylation), mixed variability and inheritance of methylation patterns, wherein it is taken into account that sequence variability is unequivocally confirmed in a system insensitive to methylation.

Preferentially the analysis of the significance of individual variability types and events within regenerants and regenerant progeny plants, as grouped by the method of derivation, is performed based on qualitative matrices and quantitative matrices of events describing all theoretically possible events, as well as regenerant plants and regenerant progeny.

Preferentially the statistical analysis results for individual regenerants and bulk regenerant progenies obtained through various means are compiled into a whole; quantitative parameters are entered for variability induced by the method of derivation and of somaclonal variability for individual regenerants and bulk regenerant progenies as well as for separate regenerant progenies grouped by the method of derivation and event types along with statistical parameters.

Preferentially those event types are identified, which are rare in culture or which do not occur at all, as well as those which are particularly subject to the activity of factors inducing variability at the level of regenerant and regenerant progeny, as well as to determine their quantitative parameters.

Preferentially individual event types are studied at the molecular level through the sequence analysis of AFLP fragments representing individual types of events, and by the same token types of somaclonal variability.

Preferentially one or more bulk regenerant progeny is analyzed.

Preferentially the quantitative characteristics of an event are given for each individual regenerant progeny: sequence variability, broad sequence variability, epigenetic variability of methylation patterns *(de novo* methylation, demethylation), stability of inherited methylated and non-methylated sites (conservative), against changes induced in cultures as well as entering quantitative differences in variability between different derivation methods of plant material.

Preferentially statistical analysis is performed of the significance of individual event types among the regenerants, bulk regenerant progeny and individual regenerant progenies as needed, grouped by the method of derivation of the research material. Preferentially the somaclonal variability level of individual plants which are regenerant progeny is determined independenly, using the principles used for somaclonal variability.

Preferentially the hypotheses relating to the sources of variability induced by the method of derivation of regenerants and that inherited as a result of generative propagation are tested.

Preferentially the detailed analysis is performed of the causes of sequence variability after introducing minor AFLP procedural changes, based on the selective amplification of research material using a tagged primer complementary to conservative sites of transposable elements, or to macrosatellite sequences.

Preferentially the segregation of genetic and epigenetic markers are analyzed for mapping populations derived through plant-plant crosses which are the progeny of regenerants with a known genotype, based on AFLP profiles for both AFLP systems. Preferentially the method is designed for deriving plants *in vitro* with a lowered or enhanced level of somaclonal variability through the identification of the causes of this variability.

Preferentially the genetic and epigenetic purity of arbitrary material derived through *in vitro* culture is verified.

Preferentially when the method is used in the analysis of the inheritance of epigenetic variability during sexual crossing.

Preferentially the method is a tool for the identification of epigenetic markers linked with the utilitarian characteristics of the plant material.

Preferentially the method is used for the identification of differences in DNA methylation patterns for various tissues of an individual plant.

The attached figures facilitate a better description of the nature of the present invention.

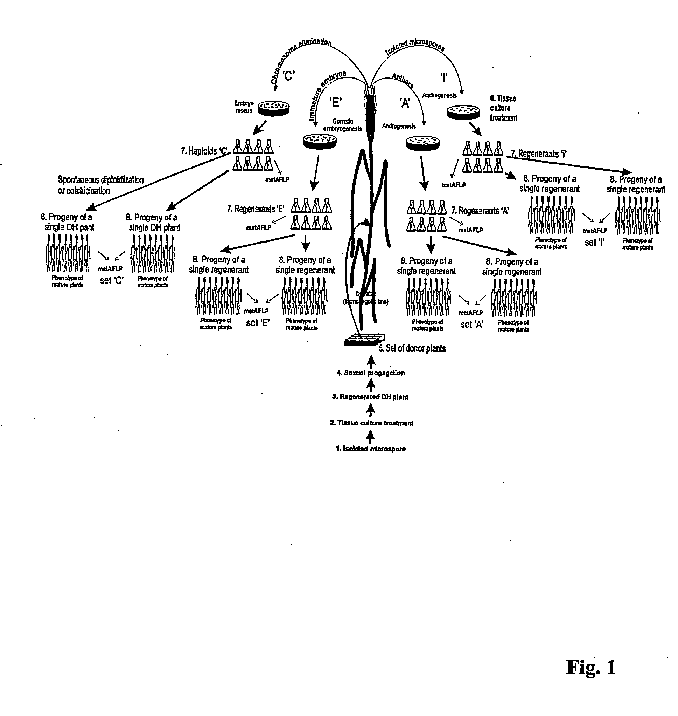

Figure 1 represents general scheme for the evaluation of plant materials to study tissue culture induced (level 7) and somaclonal variation (level 8) due to regeneration method (level 6). Generative reproduction induced variation and purity of the initial material along with the reproducibility of the AFLP approaches are verified at 5. In the described example regenerants and donor plants.were analyzed (level 7) following embryo- and androgenesis paths only.

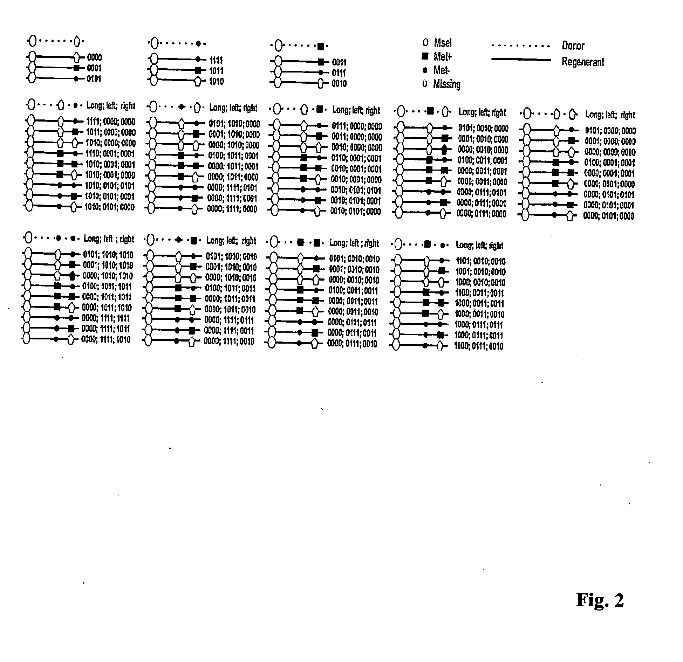

Figure 2 represents arrangements of the theoretical donor and regenerant genotypes and AFLP patterns putatively amplified by *Acc65*I/*Mse*I and *Kpn*I*lMse*I digests. The first and the third number reflect donor and the second and fourth numbers regenerant profiles; the first two numbers depict *Acc65*I/*Mse*I and the remaining *Kpn*I/*Mse*I digests. 1 and 0 state for presence and absence of a band on a profile.

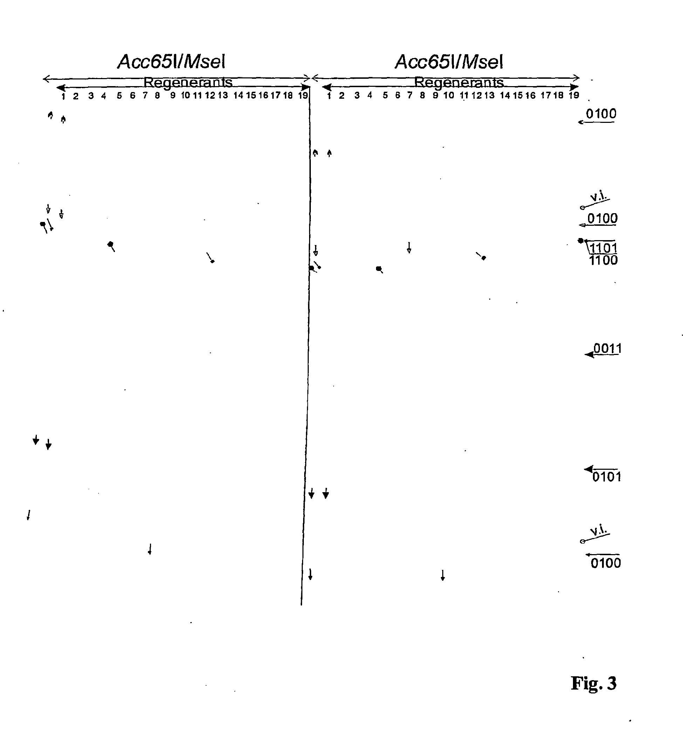

Figure 3 represents *Acc65*I*lMse*I and *Kpn*I*lMse*I metAFLP fingerprints of the regenerants (lines 1-19) and donor (D) plant, respectively; lanes 1-7 - regenerants obtained via embryogenesis; lanes 8-19 - regenerants obtained via androgenesis (see
Figure 1); different types of arrows indicate some of the events described as present/absent "1-0" according to the Fig. 7; v.i. - band of variable intensity.
Figure 4 represents Clustering of the *Acc56*I/*Mse*I and *Kpn*I*lMse*I data. 'a-r' - states for *Acc65*I/*Mse*I and 'k-r' for *Kpn*I*lMse*I*,* numbers following 'a-r' or 'k-r' reflect the given regenerated plant. 'Aparent' and 'Kparent' state for donor plant.
Figure 5 represents factorial analysis of the fingerprint data generated by *Acc65*I/*Mse*I and *Kpn*I/*Mse*I digests based on DNAs of the regenerated plants.

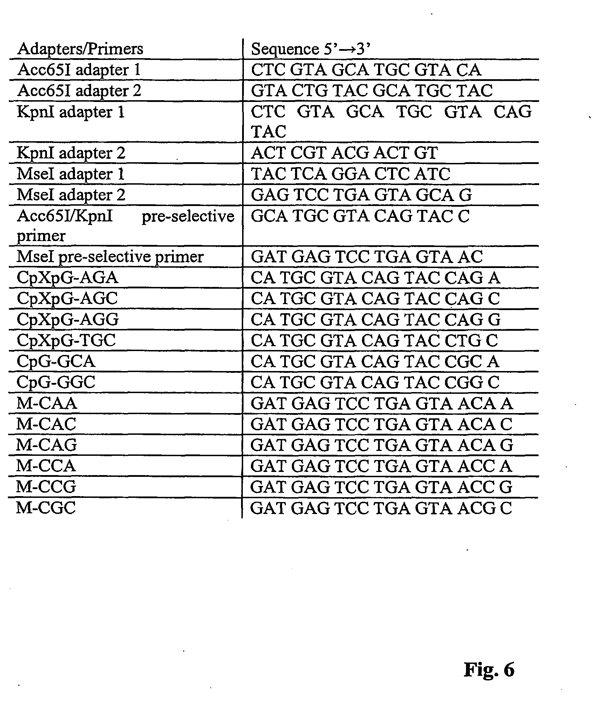

Figure 6 represents primer sequences for metAFLP.
Figure 7 represents variants detected by metAFLP. *Acc65*I/*Mse*I and *Kpn*I/*Mse*I enzyme pairs were used for the digestion of templates; 1/0 - AFLP band is present/missing. AFLP pattern column: the first two figures state for *Acc65*I/*Mse*I and the reminder for *Kpn*I*lMse*I digests; first and third positions reflect donor and second and fourth regenerant profiles. Column "Event type" classifies the given pattern to the event type. Additionally, the information is given whether the pattern is unequivocal. E, A and S state for embryo-, androgenesis-derived regenerants and all regenerants taken together expressed as absolute and normalized (%) values. Calculations of the tissue culture induced variation are based on event types.

Example embodiments of the present invention defined above are presented below.

### Plant material

A single double haploid barley plant, obtained via androgenesis from an isolated microspore (Oleszczuk, et al. 2005) was self-fertilised. A set of its sixteen progeny plants that did not differ in phenotypes was grown under controlled conditions (16h/8h day/night regime, light intensity 27 µmolm⁻²s⁻¹ and day/night temperatures of, respectively, 10°C and 12°C). The set was used to test cryptic segregation due to sexual propagation at the molecular level. After reaching proper developmental stage one progeny plant - donor one (Fig 1 general scheme) was exploited as a source of immature embryos and anthers. Somatic embryogenesis was induced from immature embryo-derived calli (Zimny and Lörz 1989) grown for three weeks on semi-solid N6 medium (Chu 1978) containing 2mgl⁻¹ 2.4-D. Somatic embryos were excised from callus, and transferred to a regeneration medium containing 0.4µM kinetin in order to encourage their germination. Emerging plantlets were transferred to Erlenmeyer flasks until they reached a height of 10-12cm, after which they were potted, acclimatized for two weeks, and finally grown under standard greenhouse conditions to maturity. To induce androgenesis, tillers of the donor plant were harvested when the microspores were between the uninucleate and the two-nucleate pollen stage. The cut ends of the tillers were held in water under low light intensity for 7 days at 4°C. The spikes were then surface sterilized by dipping first in 70% ethanol and then in 10% sodium hypochloride, after which they were rinsed five times in sterile water. Anthers were excised, placed on semi-solid N6 medium (Chu 1978) supplemented with 2mgl⁻¹ 2,4-D and 0.5µM kinetin, and cultured in 6cm diameter Petri dishes in the dark at 26°C. Embryogenic calli were transferred to 190-2 regeneration medium (Pauk, et al. 1991). Developed androgenic embryos were transferred onto fresh 190-2 medium (Zhuang and Xu 1983) supplemented with hormones according to Pauk (Pauk, et al. 1991) and solidified with 5g/l Phytagel, and kept under a 16h/8h day / night regime. Plantlets were rooted in glass tubes, and later potted, transferred to a growth chamber for a short acclimatization period, and grown to maturity in a controlled environment chamber. Before anthesis, spikes of each regenerant plant were bagged to ensure self-pollination. The two sets of regenerants, obtained via somatic embryogenesis (Set: Regenerants 'E'), and via androgenesis (Set: Regenerants 'A' level 7, Figure 1), along with that of the donor plant, provided the material to study tissue culture-induced variation.

### DNA manipulation

Total genomic DNA was isolated from approximately 100mg of 7-day-old seedling leaf using the DNeasy MiniPrep Kit (Qiagen). metAFLP oligonucleotides were obtained from the Centre for Macromolecular Studies of the Polish Academy of Sciences (Lódź) (sequences listed in Table 1). The AFLP procedure followed Vos, et al. (Vos, et al. 1995) with minor modifications (Bednarek, et al. 2002), using the restriction enzyme pairs *Acc651*I/*Mse*I and *Kpn*I*lMse*I for the initial digestion of genomic DNA. About 500ng of genomic DNA was digested with *Acc65*I/*Mse*I and a similar quantity with *Kpn*I*lMse*I at 37°C for 3h, stopping the reaction with an incubation of 15min incubation at 70°C. The relevant adaptors were ligated to the restriction digest in a final volume of 25µl at 20°C for 12h and diluted 1:3 in TE buffer. Non-selective PCR was performed using a standard amplification profile, in a total volume of 25µl using pre-selective primers (Table 1), and this reaction was diluted 1:20 in TE. Labelled selective PCRs were carried out in a final volume of 10µl in the presence of 5'-(³²P) labelled selective primers. Following PCR, samples were denatured by adding 6µl of 80% formamide loading buffer in the presence of bromophenol blue and xylene cyanol dyes. After a denaturation step (95°C for 10 minutes followed by quenching at 5°C), 6µl of reaction were loaded on a 7% 50cm x 0.4mm denaturing polyacrylamide gel. After electrophoresis, X-ray films were exposed to gels overnight at -70°C.

### Data scoring and analysis

XIStat-pro v.7.5.2 excel add-in software (www.x1stat.com, Addinsoft) was used to perform clustering (UPGMA, Jaccard's coefficient) and factorial (Pearson correlation coefficient without axes rotation) analyses. Estimates of similarity were based on Jaccard's coefficient and clustering was performed by UPGMA (Saiotu and Nei 1986). Analysis of molecular variance (AMOVA) and the Mantel test was carried out using GeneAlex5.1 excel add-in software (Peakall and Smouse 2001). The analytical treatment of the data is explained in the context of the results themselves (see below, Table 2). Kruskal-Wallis statistics (XIStat-pro v.7.5.2) were used to test whether the extent of tissue culture induced variation differed significantly between the embryo- and androgenesis-derived regenerants and among all regenerants.

### Results

### Testing the uniformity of the sexually-derived progeny

AFLP templates of the sixteen sexually derived plants were generated from both *Acc65*I/*Mse*I and *Kpn*I/*Mse*I digests, and amplified with ten selective primer combinations (CpG-GGC/MCAA; CpG-GGC/MCAC; CpG-GGC/MCAG; CpXpG-AGA/MCAC; CpXpG-AGA/MCCA; pXpG-AGC/MCAC; CpXpGAGC/MCGA; CpXpG-AGC/MCGC; CpXpG-AGG/MCAA; CpXpG-AGT/MCGC) to produce a total of 764 fragments over both digests. The profiles were invariant across all sixteen plants, except in the cases of selective primer combination (CpG-GGC/MCAC) which delivered two variable *Acc65*I/*Mse*I fragments, and CpXpG-AGT/MCGC, which produced one variable *Kpn*I*lMse*I fragment. Thus the background rate of variation is of the order of 0.26% in *Acc651*/*MseI* and 0.13% *KpnIlMseI.* When recalculated on the basis of the number of events (total number of polymorphic signals that differed from the most representative 0 or 1 signals divided by the number of plants and multiplied by number of the scored AFLPs), we conclude that the sexual mode of reproduction generates polymorphism at a frequency of 0.03 to 0.04%.

### Testing embryo and androgenesis-derived regenerants

In all, 19 independent viable plants were regenerated from tissue culture. These consisted of seven in set E and twelve in set A. All plants were fully self-fertile and resembled their mother plant in phenotype. Uniform and reproducible fingerprints were generated from the templates of the parental donor and its regenerants using seven selective primer pairs (CpXpGAGG/CAG; CpG-GCA/MCGC; CpXpG-AGC/MCCA; CpXpG-AGG/MCAC; CpGGGC/MCAA; CpXpG-TGC/MCCG; CpXpG-AGA/MCAA) (Figure 3). A total, 272 fragments were identified across both the *Acc65*I/*Mse*I and *Kpn*I*lMse*I AFLP profiles. In each of the sets of profiles, 229 fragments were invariant within the test DNA set (all regenerants and the donor plant). In all, 206 fragments were monomorphic across all samples. *Acc65*I and *Kpn*I each identified 43 polymorphic fragments, of which 23 were specific to, but monomorphic within one of the isoschizomer combinations. Among these, eight *Acc65*I/*Mse*I and seven *Kpn*I*lMse*I fragments varied only with respect to band intensity.

### Statistical analysis

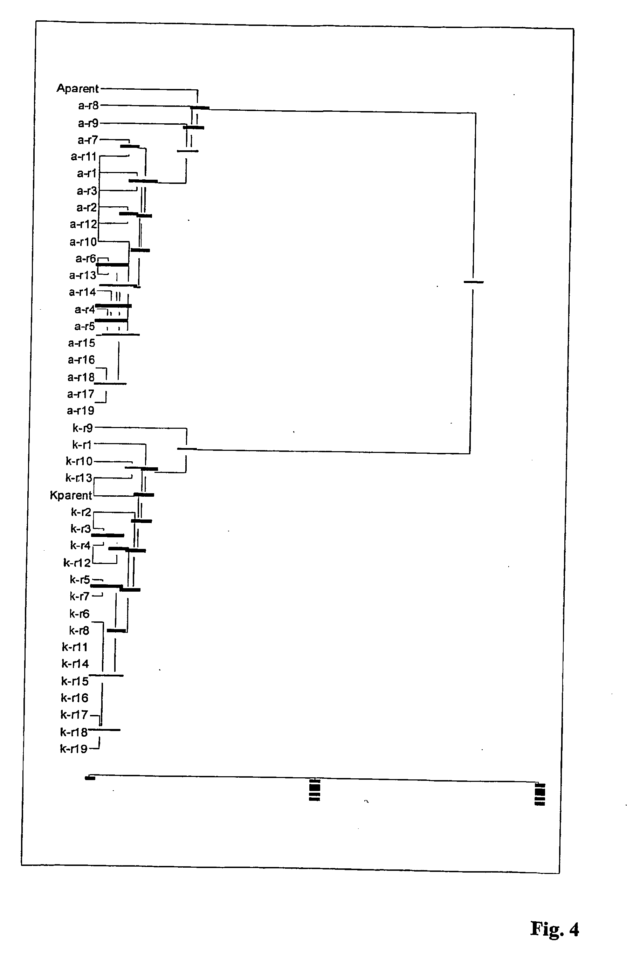

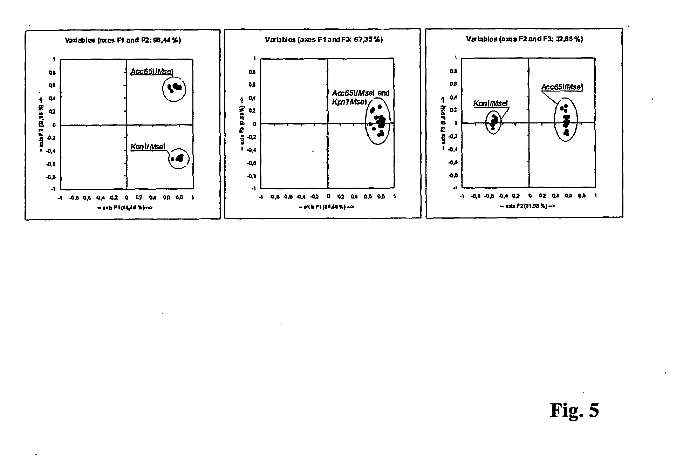

Cluster analysis demonstrated that the DNAs of the regenerated plants digested with *Acc65*I/*Mse*I formed clusters that were distinct and independent from those digested with *Kpn*I*lMse*I*.* No individual subclusters for sets 'E' or 'A' were evident (Figure 4). A factorial analysis (convergence 0.001 after 25 iterations) showed that variables F1 and F2 explain 98% of the variation identified between the *Acc65*I/*Mse*I and *Kpn*I*lMse*I sets. The respective proportions for F1-F3 and F2-F3 were 67% and 33%. Once again two clusters were formed, with the *Kpn*I*lMse*I group being more compact than the *Acc65*I/*Mse*I one (Figure 5). AMOVA showed that 69% of the variation resulted from differences between the two digests, but nonetheless the data sets remain highly correlated, as indicated by the Mantel test (R²>0.9991, p=0.001).

### Explanation of metAFLP patterns

The experimental system was designed to detect a number of distinct events that may have occurred during tissue culture. The isoschizomers recognize the same cleavage sequence, but differ in their sensitivity to methylation at the recognition site: *Acc65*I digests non-methylated, but not methylated sequences, whereas *Kpn*I is insensitive to recognition site methylation. The various changes detectable by this metAFLP approach are summarized in Table 2, in which regenerant profiles are compared to those of the donor. Most (seven) of the patterns could have been generated by a single event, although for some variants more than one explanation is possible. For example, the '0011' pattern describes a fragment that is methylated in both the donor and the regenerant template, so that it is present in the *Kpn*I/*Mse*I*,* but absent in the *Acc56*I*lMse*I digest, and is not a tissue culture-induced event; while the '0101' pattern is delivered where a tissue culture-induced event introduces a non-methylated recognition site not present in the parental template. In contrast, the '1000' pattern could have been generated either from the de-methylation of a single methylated site or the de-methylation of the external site (if two adjacent sites are considered). Some of the patterns could not be assigned to a single event ('0010' or '0001') and these are referred to as 'mixed variation'. All possible donor - regenerant pairs are given in figure 2 and permissible classification of the AFLP pattern to the appropriate event type.

### Tissue culture-induced variation

Among the regenerants, 11 out of the 14 possible patterns described in Table 2 were observed at least once. Two mixed (0100 and 1001) and a single sequence variation (0010) patterns were not detected. Across all the regenerants, 94.7% of the restriction fragments were conserved with respect to methylation status, 95.1 and 94.4% were conserved for the embryo- and anther-derived plants. Of these invariant sequences, non-methylated recognition sites were much commoner than methylated ones. Our estimation of tissue culture-induced event frequency was based on the following reasoning: first, the total number of events (5168) was taken as the number of analyzed *Acc65*I/*Mse*Iand *Kpn*I*lMse*I fragments (272) times the number (19) of regenerants. The equivalent numbers for the E and A regenerants were, respectively, 7x272 (1904) and 12x272 (3264). Distinct tissue culture-induced events were identified by scoring for the presence/absence of fragments in the two digests, across the donor and the regenerant DNAs, as detailed in Table 2 (If the band was missing in one of the digests and present in the second in at least case then it was scored as zero and one, respectively, resulting in two 0-1 matrices of the same dimension). The total number of changes was equal to the whole number events less that of the methylation inheritance (0011 and 1100 patterns). This value equals the number of events comprising total tissue culture-induced variation. The frequency of changes in *Kpn*I*lMse*I digest was calculated as the total number of differences among donor and regenerant profiles and gives an estimate of sequence variation. Then, the frequency of tissue culture-induced site methylation is the difference between that for total tissue culture-induced variation and that identified in the *Kpn*I*lMse*I digests. These are converted to percentages by using the appropriate total number of fragments surveyed (5168, 1904 and 3264 for total, E and A). For example, the total number of changes identified based on both digests is 275 (5.3%), the number of sequence changes 101 (2.0%), and the number of methylation variants 174 (3.4%). This last estimate comprises percentages of total tissue culture induced variation (TTCV) less than percentages of sequence variation (SV) calculated based on *Kpn*I*lMse*I digest. Calculation of the TTCV is the sum of 11 events (Table 2) normalized by the number of events and expressed in percentages. Conserved sites (those that did not change methylation status) were defined in percentage terms as all scored sites less TTCV varied sites (94.7% across all regenerants). Inherited methylation sites were scored as all the sites that did not change methylation status (remaining either methylated or non-methylated). These represent 12.7% of fragments. The frequency of de-methylation is of the same level in somatic and in gametic cells (0.4%). In contrast, *de novo* methylation occurred a bit less frequently in 'E' (1.1%) than in 'A' (1.4%). Our estimate of the overall frequencies of methylation events over the two regenerant types is, respectively, 3.0% and 3.6%. 'Mixed events' accounted for 1.5% and 2.0%, respectively. Similarly, the frequencies of sequence variation were 1.9% and 2.1%. Finally, the overall proportions of restriction fragments altered as a consequence of the tissue culture treatment were 4.9% and 5.6% for, respectively, the E and A regenerant.

All the regenerated plants displayed either sequence variation or/and changes in methylation pattern, with a mean of about 14 alterations per plant. About 44% of the changes affected the embryogenesis-derived plants. There was little difference in the mean number of sequence changes and alterations in methylation patterns between the embryo- and the anther-derived plants. The tissue culture-induced variation was, however, unevenly distributed among the regenerants, varying from 10 to 16 events in the former, and from 13 to 20 in the latter regenerant types. The Kruskal-Wallis statistics, however, suggest that there is no significant effect of regeneration mode on the extent of tissue culture-induced variation.

### Hotspots of tissue culture induced variation

We observed eight fragments which appear to have behaved as hotspots of variation, in the sense that all or nearly all of the regenerated plants were altered in sequence or in methylation status in relation to the donor. Thus for example, the *Acc65*I profile of all regenerants included a fragment that was absent in the donor, while the same fragment was present in the *Kpn*I profiles of both donor and regenerant. The events were classified as mixed (0100 -100%; two 1000 - 100%;), de-methylation (0111 - 100%), de novo methylation (1011 - 95%) and sequence change and three sequence (0001-100%; 0001 - 95%; 1010 - 95%) events.

An important finding is that nearly 95% of sites were faithfully transmitted to all the regenerant plants independently of how they were regenerated (via embryo or androgenesis). Interestingly, only ca. 12% of the sites remain stably methylated which is nearly seven times less than non-methylated ones. This data is comparable with that of (Chakrabarty, et al. 2003) who assessed the extent and pattern of cytosine methylation during somatic embryogenesis in *Eleurenococcus senticosus,* and showed that over 11% of sites were methylated in embryogenic callus tissue. Rather higher estimates of the extent of methylation have been obtained in rice (16%), and in embryo-derived oil palm (20%) by (Jaligot, et al. 2000).

The strategy we have developed has allowed us to both quantify and qualify the tissue culture-induced variation in barley plants regenerated from embryos and microspores. We show that this occurs at a frequency of two magnitudes higher than the background rate associated with sexual reproduction, and have been able to partition the variation into changes in methylation state and sequence, to estimate the proportion of sites unaffected by variation, to distinguish between methylated and non-methylated sites, and to predict the appearance of bands with varying intensity. We may also examine changes that affected individual plants and specify the degree they may differ from the donor one. Although not attempted here we believe that by using specially designed selective primers it should be possible to differentiate among various types of methylation and thereby obtain some insight into sequence-variation defining whether it is induced by migration of mobile elements, by changes in microsatellite copy number or by mutations within functional genomic regions belonging to different protein families. We believe that our analytical approach is the first which can differentiate between the two major classes of tissue-induced epigenetic (methylation) and genetic (sequence mutation) variation, and provide a quantitative and qualitative description of their relative contribution. This represents a means to access tissue-induced variation among regenerants sharing a uniform phenotype. Moreover, after minor modifications the approach could be used to study inheritance of the tissue culture induced variation among progenies of the regenerants giving characteristics of somaclonal variation. It is possible to anticipate the development of this approach for the dissection of epigenetic inheritance.

### REFERENCES

Aina R, Sgorbati S, Santagostino A, Labra M, Ghiani A, Citterio S. (2004) Specific hypomethylation of DNA is induced by heavy metals in white clover and industrial hemp. Physiol Plant 121:472-480.
Arene L, Pellegrino C, Gudin S. (1993) A comparison of the somaclonal variation level of Rosa hybrida L. cv. Meirutral plants regenerated from callus or direct induction from different vegetative and embryonic tissues. Euphitica 71:83-90.
Bednarek PT, Kubicka H, Zawada M. (2002) Morphology, cytology and BSA based testing on limited segregation population AFLPs. Cell Mol Biol Let 7:635-648.
Bouman H, De Klerk G-J. (2001) Measurement of the extent of somaclonal variation in begonia plants regenerated under various conditions. Comparison of three assays. Theor Appl Genet 102:111-117.
Brar DS, Jain SM. (1998) Somaclonal variation: mechanism and applications in crop improvement. In: Somaclonal variationa and Induced Mutations in Crop Improvement. Kluwer Academic Publishers, Dordrecht, Boston, London:16-37.
Bregitzer P, Halbert SE, Lemaux PG. (1998) Somaclonal variation in the progeny of transgenic barley. Theor Appl Genet 96:421-425.
Breyne P, Rombaut D, Gysel A, Montagu M, Gerats T. (1999) AFLP analysis of genetic diversity within and between Arabidopsis thaliana ecotypes. Mol Gen Genet 261:627-634.
Brown PTH, Lange FD, Kranz E, Lörz H. (1993) Analysis of single protoplasts and regenerated plants by PCR and RAPD technology. Molecular General Gentics 237:311-317.
Cervera M. Ruiz-Garcia L, Martinez-Zapater J. (2002) Analysis of DNA methylation in Arabidopsis thaliana based on methylation-sensitive AFLP markers. Molecular Genetics and Genomics 268:DOI 10.1007/s00438-002-0772-4.
Chakrabarty D, Yu KW, Paek KY. (2003) Detection of DNA methylation changes during somatic embryogenesis of Siberian ginseng (Eleuterococcus senticosus). Plant Science 165:61-68.
Chu C. (1978) The N6 medium and its applications to anther culture of cereal crops. Proc. Symp. Plant Tissue Culture, Science Press Beijing:43-50.
Devaux P, Kilian A, Kleinhofs A. (1993) Anther culture and Hordeum bulbosum-derived barley doubled haploids: mutations and methylation. Mol. Gen. Genet. 241:674-679.
Donini P, Elias ML, Bougourd SM, Koebner ROD. (1997) AFLP fingerprinting reveals pattern differences between template DNA extracted from different plant organs. Genome 40:521-526.
Fourré J-L, Berger P, Niquet L, André P. (1997) Somatic embryogenesis and somaclonal variation in Norway spruce: morphogenetic, cytogenetic and molecular approaches. Theor Appl Genet 94:159-169.
Freyssinet M, Freyssinet G. (1988) Fertil plant regeneration from sunflower (Heliantus annuus L.) immature embryos. Plant Sci. 56:177-181.
Gaj MD. (2001) Direct somatic embryogenesis as a rapid and efficient system for in vitro regeneration of Arabidopsis thaliana. Plant Cell Tiss Org 64:39-46.
Gray DJ, Compton ME, Harrell RC, Contliffe DJ. (1995) Somatic embryogenesis and the technology of synthetic seed. In: Biotechnology in Agriculture and Forestry. Somatic Embryogenesis and synthetic seed I. Springer, Berlin 30:126-151.
Guzy-Wróblewska J, Szarejko I. (2003) Molecular and agronomic evaluation of wheat double haploid lines obtained through maize pollination and anther methods. Plant Breeding 122:305-313.
Habu Y, Kakutani, T. and Paszkowski, J. (2001) Epigenetic developmental mechanisms in plants: Molecules and targets of plant epigenetic regulation. Curr. Opin. Genet. Dev. 11:215-220.
Hossain MA, Konisho K, Minami M, Nemoto K. (2003) Somaclonal variation of regenerated plants in chili pepper (Capsicum annuum L.). Euphytica 130:233-239.
Jacobsen SE, Sakai, H., Finnegan, E. J., Cao, X. and Meyerowitz, E. M. (2000) Ectopic hypermethylation of flower-specific genes in Arabidopsis. Curr. Biol. 10:179-186.
Jaligot E, Beulé T, Rival A. (2002) Methylation-sensitive RFLPs: characterisation of two oil palm markers showing somaclonal variation-associated polymorphism. Theor Appl Genet 104:1263 - 1269.
Jaligot E, Rival A, Beulé T, Dussert S, Verdeil J-L. (2000) Somaclonal variation in oil palm (Elaeis guineensis Jacq.): the DNA methylation hypothesis. Plant Cell Reports 19:684 - 690.
Kakutani T, Munakata, K., Richards, E. J. and Hirochika, H. (1999) Meiotically and mitotically stable inheritance of DNA hypomethylation induced by ddm1 mutation of Arabidopsis thaliana. Genetics 151:831-838.
Kudirka DT, Schaeffer GW, Beanzinger PS. (1989) Stability of ploidy in meristems of plants regenerated from anther calli of wheat (Triticum aestivum L. em. Thell.). Genome 32:1068-1073.
Larkin PJ, Scowcroft WR. (1981) Somaclonal variation - a novel source of variability from cell cultures for plant improvement. Theor Appl Genet 60:197-214.
Linacero R, Freitas AE, Vázquez AM. (2000) Hot spots of DNA instability revealed through the study of somaclonal variation in rye. Theor Appl Genet 100:506-511.
Munthali MT, Newbury HJ, Ford-Lloyd BV. (1996) The detection of somaclonal variants of beet using RAPD. Plant Cell Reports 15:474 - 478.
Oleszczuk S, Sowa S, Zimny J. (2005) Androgenic response to pre-culture stress in the microspore cultures of barley. Protoplasma in press
Pauk J, Manninen O, Mattila I, Salo Y, Pulli S. (1991) Androgenesis in hexaploid spring wheat F2 populations and their parents using a multiple-step regeneration system. Plant Breeding 107:18-27.
Peakall R, Smouse PE. (2001) GenALEx V5: Genetic Analysis in Excel. Population genetic software for teaching and research. Australian National University, Canberra. http://www.anu.edu.au/BoZo/GenAIEx/
Peraza-Echeverria S, Herrera-Valencia VA, James-Kay A. (2001) Detection of DNA methylation changes in micropropagated banana plants using methylation-sensitive amplification polymorphism (MSAP). Plant Science 161:359-367.
Peshke VM, Phillips RL. (1991) Activation of the maize transposable element Suppreassor-mutator (Spm) in tissue culture. Theor Appl Genet 81:90-97.
Portis E, Acquadro A, Comino C, Lanteri S. (2003) Analysis of DNA methylation during germination of pepper (Capsicum annuum L.) seeds using methylation-sensitive amplification polymorphism (MSAP). Plant Science 166:169-178.
Remotti PC. (1998) Somaclonal variation and in vitro selection for crop improvement. In: Somaclonal Variation and Induced Mutations in Crop Improvement. Eds. Jain, S.M., Brar, D.S., Ahloowalia, B.S.:169-201.
Reyna-López GE, Simpson J, Ruiz-Herrera J. (1997) Differences in DNA methylation patterns are detectable during the dimorphic transition of fungi by amplification of restriction polymorphisms. Mol Gen Genet 253:703-710.
Roseland CS, Espinasse A, Grosz TJ. (1991) Somaclonal variats of sunflower with modified coumarin expressionunder stress. Euphitica 54
Saiotu N, Nei M. (1986) The number of nucleotides required to determine the branching order of three species, with special reference to human - Chimpanzee-Gorilla divergence. J. Mol. Evaluation 24:189-204.
Thomas E, Bright SWJ, Franklin J, Lancaster V, Miflin BJ. (1982) Variation amongst protoplast-derived potato plants (Solanum tuberosum cv, "Maris Bar"). Theor Appl Genet 62:65-68.
Vendrame WA, Kochert G, Wetzstein HY. (1999) AFLP analysis of variation in pecan somatic embryos. Plant Cell Reports 18:853-857.
Vos P, Hogers R, Bleeker M, Lee T, Hornes M, Frijters A, Pot J, Peleman J, Kuiper M, Zauber M. (1995) AFLP: a new technique for DNA fingerprinting. Nucl Acids Res 23:4407-4414.
Wang ZY, Nagel J, Potrykus I, Spangenberg G. (1993) Plants from cell suspension-derived protoplasts in Lolium species. Plant Sci 94:179-193.
Xiong LZ, Xu CG, Saghai Maroof MA, Zhang Q. (1999) Patterns of cytosine methylation in an elite rice hybrid and its parental lines, detected by a methylation-sensitive amplification polymorphism technique. Mol Gen Genet 261:439-446.
Zhuang JJ, Xu J. (1983) Increasing differentiation frequencies in wheat pollen calus. In: Cell and Tissue Culture Techniques for Cereal Crop Improvement (Hu, H. and Vega, M.R. Eds). Science Press Beijing 431
Zimny J, Lörz H. (1989) High frequency of somatic embryogenesis and plant regeneration of rye (Secale cereale L.). Z. Pflanzenzüchtung 102:89 100.

## Claims

1. Use of the AFLP technique which is composed of the stages: isolation of genomic DNA, digestion of the genomic DNA with one pair of restriction endonucleases and digestion of another portion of the same DNA with another pair of restriction endonucleases, annealing of synthetic heteroduplexes to the free restriction ends, initial amplification of fragments obtained in such a manner by way of primary PCR, selective amplification, separation of the PCR products in a polyacrylamide gel, visual profile analysis for the method for the identification of variability induced by the derivation method in *in vitro* cultures at the level of regenerant plants, as well as variation inherited as a result of generative propagation consisting of the following stages:
1) estimation of the repeatability of the two AFLP technique variants: repeatability control of A FLP based on the identity of several DNA samples of the same plant through the use of AFLP and isoschizomers both sensitive and insensitive to methylation at the restriction-site and/or adjacent areas as well as a representative number of pairs of selective primers identical in all AFLP analyses;
2) estimation of the repeatability of the two AFLP technique variants: repeatability control of AFLP based on the identity of several DNA samples of the same plant through the use of AFLP and isoschizomers both sensitive and insensitive to methylation at the restriction-site and/or adjacent areas as well as a representative number of pairs of selective primers using a different set of selective AFLP primers than those used in the other AFLP analyses;
3) identification of variability induced by the method of derivation of the regenerants in relation to the donor plant is performed in two AFLP variants using isoschizomers both sensitive and insensitive to methylation at the restriction-site and/or adjacent areas as well as a representative number of pairs of selective primers identical in all AFLP analyses;
4) the identification of the tissue culture induced variability among regenerants in relation to the donor plant is performed in two AFLP technique variants utilising isoschizomers both sensitive and insensitive to methylation at the restriction-site and/or adjacent areas as well as a representative number of pairs of selective primers identical in all AFLP analyses;
5) the identification of somaclonal variability among regenerant progeny in relation to the donor plant is carried out using two AFLP technique variants utilising isoschizomers both sensitive and insensitive to methylation at the restriction-site and/or adjacent areas as well as a representative number of pairs of selective primers identical in all AFLP analyses;
6) the molecular analysis of the experimental material using the AFLP technique taking into account isoschizomers both sensitive and insensitive to methylation at the restriction-site and/or adjacent areas as well as a representative number of pairs of selective primers identical in all AFLP analyses is comprised of the following stages:
a) analyses of AFLP patterns of donor plants with respect to genetic and epigenetic variability induced during the generative propagation of an individual plant, a control of generative propagation,
b) analyses of AFLP patterns for the control of the reproducibility of both AFLP technique variants,
c) analysis of AFLP patterns for regenerants in comparison to donor plants or separate regenerants in relation to appropriate donor plants;
d) danalyses of AFLP patterns for donor plant, its regenerant and regenerant progeny, or distinct regenerant progeny lines along with appropriate donor and its regenerant plants;
7) identification of the 14 types of events recognized at the binary matrix level for systems sensitive and insensitive to methylation, which are the result of a comparison between the donor plant with regenerants and between donor plant with regenerant progeny simultaneously in two AFLP systems, wherein the identification of event types is carried out individually for each regenerant and as bulk for regenerant progenies and is represented as 14 binary matrices which describe each of the events independently, subdivided into methods of derivation; wherein the columns represent consecutive regenerants or bulks and rows represent consecutive AFLP fragments;
8) assignment of certain event types to a given type of variability induced the method of derivation in relation to each regenerant;
9) identification of the 14 types of events recognized at the binary matrix level for systems sensitive and insensitive to methylation, which are the result of a comparison between the donor plant with regenerant simultaneously in two AFLP systems;
10) identification of the 14 types of events recognized at the binary matrix level for systems sensitive and insensitive to methylation, which are the result of a comparison between the donor plant with regenerant progeny simultaneously in two AFLP systems, where the identification of event types is performed through the summation of the genotypes of a given summary progeny, and through the comparison of such a total AFLP profile of regenerant progeny with the donor plant profile,
11) the bulk regenerant progeny genotype expressed via an AFLP profile for systems sensitive and insensitive to methylation at the level of individual AFLP fragments has a different value from that of the donor plant in a given AFLP system, so long as a given fragment within it is polymorphic, or is totally different from this value for the donor plant; in the opposite case the values for the donor plant and bulk regenerant progeny for a given AFLP fragment in a given AFLP system are identical,
12) production of a quantitative matrix for regenerants, where the columns contain data for consecutive regenerants grouped by the method of derivation, and the rows contain the 14 types of events which may be assigned to each regenerant progeny;
13) production of a quantitative matrix for regenerant progeny, where the columns contain bulk data for regenerant progeny grouped by the method of derivation, and the rows contain the 14 types of events which may be assigned to each bulk regenerant progeny;
14) production of a quantitative matrix for events in regenerants, where this is a quantitative matrix for post-transposition regenerants, where the columns contain 14 events and the rows contain regenerants grouped by the method of derivation;
15) calculation of the total number of events based on the binary matrix for individual methods of derivation of regenerants as well as for regenerants, where the total number of events is calculated as a product of the number of AFLP fragments identified in both AFLP systems for all pairs of selective primers multiplied by the number of regenerants derived by a given method;
16) calculation of the total number of events based on the binary matrix for individual methods of derivation of regenerants as well as for regenerant progenies in bulk, where the total number of events corresponds to the number of DNA fragments identical or different in both AFLP systems for all pairs of selective primers used in the analysis;
17) calculation of the error stemming from generative propagation is carried out based on the binary matrix of donor plants and a single plant; determination of the number of events different from those in a single plant belonging to the donor plants, and the calculation of the total number of events for the donor plants;
18) calculation of the error stemming from the AFLP technique is carried out based on the binary matrix for DNA samples obtained from the same plant and subjected to the stages of the AFLP technique using isoschizomers sensitive and insensitive to methylation at the restriction site or its immediate surroundings as well as a representative number of selective starter pairs identical in all AFLP systems, where the total number of events and the number of polymorphic events are calculated, and the latter number is expressed as a percentage;
19) determination, based on binary matrices for regenerants and the donor plant, of groups of events describing: (a) sequence variation connected with any changes in DNA nucleotide sequences based on AFLP data from the system insensitive to methylation and (b) epigenetic variability, the *de novo* variability of methylation patterns, and additionally mixed variation which is sequence and methylation variability based on data for both AFLP systems;
20) determination, based on binary matrices for bulk regenerant progeny and the donor plant, of event groups describing (a) sequence variation comiected with all DNA nucleotide changes based on AFLP data from the system insensitive to methylation and (b) epigenetic variability, the *de novo* variability of methylation patterns, and additionally mixed variation which is sequence and methylation variability, and (c) inheritance of methylation patterns as related to the presence and/or lack of methylation at the restriction site or its vicinity, based on data for both AFLP systems;
21) identification of events particularly subject to the activity of factors inducing variability in *in vitro* cultures;
22) sequence analysis of individual event types and/or broad sequence variability, where variability is identified using AFLP technique variants using primers directed against conservative sequences within mobile elements, repeating sequences or the sequences of conservative gene families;
23) statistical analysis of the donor plant and its regenerants as well as regenerant progeny, and possibly distinct regenerants and regenerant progenies along with appropriate donor plants, as well as AFLP reproducibility control and generative propagation control:
a) statistical analysis of the results of AFLP using parametric analysis, Mantel's test and/or Cronbach's alpha coefficient as well as molecular variance analysis using the *Fₛ*, or *Phiₛ,* coefficients;
b) determination of the error of the tissue culture induced and somaclonal variability estimates, based on the Mantel test correlation coefficient;
c) determination of the quantitative parameters of variability induced by the method of derivation of regenerants and somaclonal variability for regenerants, individual regenerant progenies, talcing into account the method of derivation and giving their statistical significance level;
d) statistic determination of differences between individual types of events induced in *in vitro* cultures for regenerants, as well as bulk regenerant progeny, grouped by method of derivation and supplemented by their statistical significance using quantitative matrices of events;
e) statistical analysis of the comparison between variability induced by the method of derivation as well as somaclonal variation, determined through the comparison of the donor plant profile, and the bulk profile of regenerant progeny and their types: sequence variability and/or broad sequence variability, *de novo* methylation, demethylation, mixed variability, inheritance of: methylation patterns, non-methylated sites or conservative sites in regenerant progeny, grouped by method of derivation as well as a comparison by method of derivation of regenerant progeny, where quantitative matrices are used for events;
f) statistical comparisons of regenerants, and the bulk regenerant progenies for different methods of derivation for a given regenerant progeny, where quantitative matrices of the progeny are used;
g) statistical comparison of the results of the above mentioned points for individual regenerants and bulk regenerant progenies amongst one another;
h) statistical or qualitative comparison of somaclonal variation between individual plants being the generative progeny of regenerants;
i) collation of the existing results in the form of a compilation indicating quantitative characteristics of variability induced by the method of derivation determined at the regenerant level, as well as quantitative characteristics of inherited variability determined at the level of regenerant progeny.

2. Use according to Claim I, wherein the identification of the 14 types of events recognized at the level of binary matrix is selected from among the events represented in Fig. 7.

3. Use according to Claim 1, wherein to derive donor plants, a single plant is used which is a doubled haploid derived from an isolated microspore, wherein it is treated as a control of the genetic and epigenetic variability of donor plants.

4. Use according to Claim 1, wherein genetic and epigenetic variability of generatively propagated material, donor plants, is determined based on AFLP profiles obtained from restriction endonuclease isoschizomers recognizing and digesting identical sequences, but differing in their sensitivity to methylation at or about the restriction site, wherein one of the isoschizomers is insensitive to methylation.

5. Use according to Claim I, wherein in a system sensitive and insensitive to methylation no differences are observed in the AFLP profiles of donor plants, then the level of variability induced through generative propagation and identified with a given pair of selective primers is insignificant.

6. Use according to Claim I, wherein when there are differences in AFLP profiles for donor plants between a system sensitive and a system insensitive to methylation, a percentage portion of such an event is determined, along with the background level, generative propagation error.

7. Use according to Claim 1, wherein when there are differences in AFLP profiles for experimental replicants on the same DNA samples, the AFLP technique error is estimated in order to determine experimental error.

8. Use according to Claim 1, wherein the integral portion of the research material is composed of donor plants, along with a single plant, donor plant regenerants and separate regenerant progenies.

9. Use according to Claim I, wherein the donor plant along with regenerants and regenerant progeny compose the core of research material for the study of variability induced by the method of derivation or somaclonal variability, respectively.

10. Use according to Claim I, wherein the molecular analysis is performed using the AFLP technique in two variants, using isoschizomers differing in their sensitivity to methylation.

11. Use according to Claim I, wherein one of the isoschizomers is completely insensitive to methylation.

12. Use according to Claim 1, wherein in the identification of segregated PCR products, and isotope marker is used which is introduced to the 5' end of the primer complementary to the adaptor annealed to the restriction site freed by the isoschizomers.

13. Use according to Claim 1, wherein other methods of visualising AFLP fragments are used, including fluorescence.

14. Use according to Claim 1, wherein the profiles of both AFLP variants for the donor plant along with its regenerants and taking into account the method of derivation and AFLP reproducibility control are performed on identical AFLP primer pairs.

15. Use according to Claim 1, wherein AFLP profile analysis and their conversion to binary matrices is based on the fact that the AFLP profiles of both restrictase systems for all pairs of selective primers are entered as a binary matrix into a spreadsheet, where the data set obtained for one pair of selective AFLP primers is placed below the previous one such that each column represents a total binary profile for a donor plant in one AFLP system, and the subsequent columns contain analogous data for regenerants and controls, wherein data for the second AFLP system is recorded in subsequent columns, wherein the bands from one system are ordered like those from the previous system (in rows), and where a given band does not appear a zero is entered in the appropriate row of the spreadsheet.

16. Use according to Claim 1, wherein AFLP profile analysis and their conversion to binary matrices is based on the fact that the AFLP profiles of both restrictase systems for all pairs of selective primers are entered as a binary matrix into a spreadsheet, where the data set obtained for one pair of selective AFLP primers is placed below the previous one such that each column represents a total binary profile for a donor plant in one AFLP system, and the subsequent columns contain analogous data for regenerant progenies and controls, wherein data for the second AFLP system is recorded in subsequent columns, wherein the bands from one system are ordered like those from the previous system (in rows), and where a given band does not appear a zero is entered in the appropriate row of the spreadsheet.

17. Use according to Claim 1, wherein the AFLP profile for regenerant progeny is represented in the form of a bulk genotype per AFLP variant, sensitive and insensitive to methylation separately.

18. Use according to Claim 1, wherein the generative propagation control is carried out through the summation of the number of signals (events) from a single plant, where the number of changes is calculated as a percentage of the total number of events.

19. Use according to Claim 1, wherein the parametric analysis, Mantel's test correlation analysis and molecular variability analysis are performed using binary matrices of AFLP systems sensitive and insensitive to methylation for regenerants and/or regenerant progeny depending on whether the variability induced in culture or inherited by regenerant progeny are being analyzed.

20. Use according to Claim 1, wherein the factorial analysis is used as the graphical representation of the AFLP results.

21. Use according to Claim 1, wherein the identification of the 14 types of events recognized at the level of AFLP pattern and assigned to variability induced by the method of derivation is performed on the basis of AFLP patterns, wherein the donor plant profile is used along with regenerant profiles in both systems, and subsequently the profile of each regenerant is compared to the donor plant profile in order to identify these events.

22. Use according to Claim 1, wherein the identification of the 14 types of events recognized at the level of AFLP patterns and assigned to somaclonal variability is carried out on the basis of AFLP patterns, where the donor plant profile is used along with profiles in both AFLP systems, and the bulk profile of regenerant progeny is compared to the donor plant profile in order to identify event types, wherein it is assumed that in case of polymorphic AFLP fragments of the bulk regenerant progeny genotype, a value different than that of the donor plant is entered or a different value if it is different from that of the donor plant in all regenerant progenies for a given AFLP system.

23. Use according to Claim 1, wherein when the 14 event types comprise the basis for their segregation into groups: sequence variability, methylation variability (methylation and demethylation), mixed variability and inheritance of methylation patterns, wherein it is taken into account that sequence variability is unequivocally confirmed in a system insensitive to methylation.

24. Use according to Claim 1, wherein the analysis of the significance of individual variability types and events within regenerants and regenerant progeny plants, as grouped by the method of derivation, is performed based on qualitative matrices and quantitative matrices of events describing all theoretically possible events, as well as regenerant plants and regenerant progeny.

25. Use according to Claim I, wherein the statistical analysis results for individual regenerants and bulk regenerant progenies obtained through various means are compiled into a whole; quantitative parameters are entered for variability induced by the method of derivation and of somaclonal variability for individual regenerants and bulk regenerant progenies as well as for separate regenerant progenies grouped by the method of derivation and event types along with statistical parameters.

26. Use according to Claim 1, wherein those event types are identified, which are rare in culture or which do not occur at all, as well as those which are particularly subject to the activity of factors inducing variability at the level of regenerant and regenerant progeny, as well as to determine their quantitative parameters.

27. Use according to Claim I, wherein individual event types are studied at the molecular level through the sequence analysis of AFLP fragments representing individual types of events, and by the same token types of somaclonal variability.

28. Use according to Claim 1, wherein one or more bulk regenerant progeny is analyzed.

29. Use according to Claim I, wherein the quantitative characteristics of an event are given for each individual regenerant progeny: sequence variability, broad sequence variability, epigenetic variability of methylation patterns *(de novo* methylation, demethylation), stability of inherited methylated and non-methylated sites (conservative), against changes induced in cultures as well as entering quantitative differences in variability between different derivation methods of plant material.

30. Use according to Claim 1, wherein statistical analysis is performed of the significance of individual event types among the regenerants, bulk regenerant progeny and individual regenerant progenies as needed, grouped by the method of derivation of the research material.

31. Use according to Claim I, wherein the somaclonal variability level of individual plants which are regenerant progeny is determined independenly, using the principles used for somaclonal variability.

32. Use according to Claim 1, wherein the hypotheses relating to the sources of variability induced by the method of derivation of regenerants and that inherited as a result of generative propagation are tested.

33. Use according to Claim 1, wherein the detailed analysis is performed of the causes of sequence variability after introducing minor AFLP procedural changes, based on the selective amplification of research material using a tagged primer complementary to conservative sites of transposable elements, or to macrosatellite sequences.

34. Use according to Claim I, wherein the segregation of genetic and epigenetic markers are analyzed for mapping populations derived through plant-plant crosses which are the progeny of regenerants with a known genotype, based on AFLP profiles for both AFLP systems.

35. Use according to Claim 1, wherein the method is designed for deriving plants *in vitro* with a lowered or enhanced level of somaclonal variability through the identification of the causes of this variability.

36. Use according to Claim I, wherein the genetic and epigenetic purity of arbitrary material derived through *in vitro* culture is verified.

37. Use according to Claim 1, wherein when the method is used in the analysis of the inheritance of epigenetic variability during sexual crossing.

38. Use according to Claim 1, wherein the method is a tool for the identification of epigenetic markers linked with the utilitarian characteristics of the plant material.

39. Use according to Claim 1, wherein the method is used for the identification of differences in DNA methylation patterns for various tissues of an individual plant.

## Patentansprüche

1. Verwendung von der AFLP Technik, die die Etappen umfasst: die Isolation von genomischer DNA, Verdauen von genomischer DNA mit einem Paar von Restriktionsendonukleasen und Verdauen einer anderen Portion von der selben genomischen DNA mit einem anderen Paar von Restriktionsendonukleasen, Annealing von synthetischen Heteroduplexen zu den freien Restriktionsenden, Anfangsamplifikation von den Fragmenten derart durch primäre PCR erhaltene, selektive Amplifikation, Trennung der PCR-Produkte in einem Polyacrylamidgel, visuelles Profil-Analyse für das Verfahren zur Identifizierung von Variabilität durch die Ableitung Verfahren in *in vitro* Kulturen auf der Ebene der Regeneranten-Pflanzen induziert, sowie Variation infolge generatives Vermehrungsmaterial, bestehend aus den folgenden Schritten vererbt:
1) Schätzung der Wiederholbarkeit der beiden AFLP Technik Varianten: Wiederholbarkeit Kontrolle von AFLP in Anlehnung an der Identität von mehreren DNA-Proben der gleichen Pflanze durch die Verwendung von AFLP und Isoschizomere sowohl empfindlich und unempfindlich gegen Methylierung an der Restriktionsstellen und/oder angrenzenden Bereichen sowie einer repräsentativen Anzahl von Paaren von selektiven Primern identisch in allen AFLP Analysen;
2) Schätzung der Wiederholbarkeit der beiden AFLP Technik Varianten: Wiederholbarkeit Kontrolle von AFLP in Anlehnung an der Identität von mehreren DNA-Proben der gleichen Pflanze durch die Verwendung von AFLP und Isoschizomere sowohl empfindlich und unempfindlich gegen Methylierung an der Restriktionsstellen und/oder angrenzenden Bereichen sowie einer repräsentativen Anzahl von Paaren von selektiven Primern mit Verwendung eines anderen Satzes von selektiven AFLP-Primer als die in den anderen AFLP Analysen verwendeten;
3) Identifizierung der Variabilität durch das Verfahren der Ableitung der Regeneranten in Bezug auf der Donorpflanze induziert, wird in zwei Varianten von AFLP mit Isoschizomere sowohl empfindlich und unempfindlich gegen Methylierung an den Restriktionsstellen und/oder angrenzenden Bereichen sowie eine repräsentative Anzahl von Paaren der selektiven Primern identisch in allen AFLP Analysen durchgeführt;
4) die Identifizierung der Gewebekultur induziert Variabilität unter Regeneranten in Bezug auf der Donorpflanze ist in zwei Varianten der AFLP-Technik unter Verwendung der Isoschizomeren sowohl empfindlich und unempfindlich gegen Methylierung an der Restriktionsstellen und/oder angrenzenden Bereichen sowie einer repräsentativen Anzahl von Paaren selektive Primer identisch in allen AFLP Analysen durchgeführt;
5) die Identifizierung von der somaklonalen Variabilität unter Regeneranten Nachkommenschaft in Bezug auf dem Donorpflanze in zwei Varianten AFLP-Technik unter Verwendung der Isoschizomeren sowohl empfindlich und unempfindlich gegen Methylierung an der Restriktionsstellen und/oder angrenzenden Bereichen sowie einer repräsentativen Anzahl von Paaren selektiv Primer identisch in allen AFLP Analysen durchgeführt;
6) die molekulare Analyse der experimentellen Material mit der AFLP-Technik unter Berücksichtigung der Isoschizomeren sowohl empfindlich und unempfindlich gegen Methylierung an der Restriktionsstellen und/oder angrenzenden Bereichen sowie einer repräsentativen Anzahl von Paaren von selektiven Primer identisch in allen AFLP Analysen setzt sich zusammen aus den folgenden Schritten:
a) Analysen der AFLP-Mustern von der Donorpflanzen in Bezug auf genetische und epigenetische Variabilität während der generativen Vermehrung eines einzelnen Pflanze induziert, eine Kontrolle der generativen Vermehrung,
b) Analyse der AFLP-Mustern für die Steuerung der Reproduzierbarkeit der beiden AFLP-Technik Varianten,
c) Analyse der AFLP-Mustern für den Regenenanten im Vergleich zur Donorpflanzen oder getrennte Regeneranten in Bezug auf der geeigneten Donorpflanzen;
d) Analyse der AFLP-Mustern für die Donorpflanze, ihre Regenenant und Nachkommen von Regeneranten oder unterschiedliche Nachkommen von Regenerierungslinien zusammen mit den entsprechenden Donor und seiner Regenerierungspflanzen;
7) Identifizierung der 14 Arten von Ereignissen in der binären Matrix Ebene für Systeme empfindlich und unempfindlich gegen Methylierung, die das Ergebnis eines Vergleichs zwischen der Donorpflanze mit Regenenranten und zwischen der Donorpflanze mit Nachkommen von Regeneranten gleichzeitig in zwei AFLP Systeme erkannt wird, wobei die Identifizierung von Ereignistypen wird individuell für jeden Regenenranten und als Masse den Nachkommen von Regeneranten durchgeführt und wird als 14 Binärmatrizen die jedes der Ereignisse unabhängig beschreiben dargestellt, unterteilt in Methoden der Ableitung, wobei stellen die Spalten die folgenden Regeneranten oder Massen und Zeilen die folgenden AFLP-Fragmente dar;
8) Zuordnung bestimmter Ereignistypen zu einer bestimmten Art von Variabilität induziert das Verfahren der Ableitung in Bezug auf jede Regenerant;
9) Identifizierung der 14 Arten von Ereignissen in der binären Matrix Ebene für Systeme empfindlich und unempfindlich gegen Methylierung, die das Ergebnis eines Vergleichs zwischen der Donorpflanze mit Regeneranten gleichzeitig in zwei Systemen AFLP erkannt werden;
10) Identifizierung der 14 Arten von Ereignissen in der binären Matrix Ebene für Systeme empfindlich und unempfindlich gegen Methylierung, die das Ergebnis eines Vergleichs zwischen der Donorpflanze mit Regeneranten Nachkommen gleichzeitig in zwei AFLP Systemen sind, wobei die Identifizierung von Ereignistypen durch die Summe der Genotypen einer bestimmten Nachkommenzusammenfassung, und durch den Vergleich eines solchen gesamten AFLP Profils von Regeneranten-Nachkommen mit dem Donorpflanzen-Profil durchgeführt wird,
11) die Masse von Regeneranten-Nachkommen Genotyp über ein AFLP Profil von Systemen empfindlich und unempfindlich gegen Methylierung auf der Ebene der einzelnen AFLP-Fragmente exprimiert, hat einen anderen Wert als diese von der Donorpflanze in einer bestimmten AFLP-System, solange innerhalb einer bestimmten Fragment ist polymorphen oder ist völlig verschieden von diesem Wert für die Donorpflanze, im umgekehrten Fall sind die Werte für die Donorpflanze und Massen-Regeneranten Nachkommen für eine gegebene AFLP Fragment in einem gegebenen System AFLP identisch,
12) Herstellung einer Matrix für die quantitative Regeneranten, wobei die Spalten enthalten die Daten für aufeinanderfolgende Regeneranten durch das Verfahren der Ableitung gruppiert, und die Zeilen enthalten die 14 Arten von Ereignissen, die zu jedem Regeneranten-Nachkommen zugewiesen werden kann;
13) Herstellung einer Matrix für die quantitative Regeneranten-Nachkommen, wobei die Spalten enthalten der Datenblock Regeneranten-Nachkommen durch das Verfahren der Ableitung gruppiert, und die Zeilen enthalten die 14 Arten von Ereignissen, die zu jedem Massen-Regeneranten-Nachkommen zugewiesen werden kann;
14) Herstellung einer quantitativen Matrix für Ereignisse in Regeneranten, wo das ist eine quantitative Matrix für post-Umsetzung Regeneranten, wo die Spalten enthalten 14 Veranstaltungen und die Zeilen enthalten Regeneranten nach der Methode der Ableitung gruppiert;
15) Berechnung der Gesamtzahl der Ereignisse basierend auf der binären Matrix einzelner Methoden zur Ableitung von Regeneranten als auch für Regeneranten, wobei die Gesamtzahl der Ereignisse berechnet als ein Produkt der Anzahl von AFLP-Fragmente identifiziert in beiden AFLP-Systeme für alle Paare der selektiven Primersequenzen durch die Anzahl der Regeneranten von einem bestimmten Verfahren abgeleitet multipliziert;
16) Berechnung der Gesamtzahl der Ereignisse basierend auf der binären Matrix einzelner Methoden zur Ableitung von Regeneranten als auch für Regeneranten-Nachkommen in der Masse, wobei die Gesamtzahl der Ereignisse entspricht der Anzahl von DNA-Fragmenten gleich oder verschieden in beiden AFLP für alle Paare von selektiven Primer in der Analyse verwendet;
17) Berechnung der Fehler, die aus generatives Vermehrungsmaterial resultieren, wird auf der Grundlage der binären Matrix Donorpflanzen und einer einzigen Pflanze durchgeführt; die Bestimmung der Anzahl von Ereignissen, die sich von denen in einer einzigen Pflanze von der den Donorpflanzen unterscheiden, und die Berechnung der Gesamtzahl der Ereignissen für die Donorpflanzen;
18) Berechnung der Fehler, die aus der AFLP-Technik resultieren, wird auf der Grundlage der binären Matrix für DNA-Proben erhaltenen aus der gleichen Pflanze und ausgesetzten der Stufen der AFLP-Technik unter Verwendung der Isoschizomeren empfindlich und unempfindlich gegen Methylierung an der Restriktionsstelle oder seiner unmittelbaren Umgebung als auch eine repräsentative Anzahl von selektiven Starter Paaren identisch in allen AFLP Systemen durchgeführt, wobei die Gesamtzahl der Ereignisse und der Anzahl der polymorphen Ereignisse berechnet werden, und diese Zahl wird als Prozentsatz ausgedrückt;
19) Bestimmung, basierend auf Binärmatrizen für Regeneranten und Donorpflanze, von Gruppen von Ereignissen, die beschreiben: (a) Sequenzvariation verbundene mit allen Änderungen in der DNA-Nukleotid-Sequenzen basierende auf AFLP Daten aus dem System unempfindlich gegenüber Methylierung und (b) die epigenetische Variabilität, die Variabilität der *de novo* Methylierungsmuster, und zusätzlich gemischte Variabilität, die ist Sequenz und Methylierung Variabilität basierend auf AFLP Daten für beide Systeme ist;
20) Bestimmung, basierend auf Binärmatrizen für Massen-Regenerante-Nachkommen und dem Donorpflanze, der Gruppen von Ereignissen beschreiben (a) Sequenzvariation verbunden mit allen DNA-Nukleotid-Änderungen basierend auf AFLP Daten aus dem unempfindlichen gegenüber Methylierung System und (b) epigenetische Variabilität, die *de novo* Variabilität der Methylierungsmustern und zusätzlich gemischte Variation der Sequenz und Methylierung Variabilität, und (c) Vererbung von Methylierungsmustern in Verbindung mit der Anwesenheit und/oder Mangel von Methylierung an der Restriktionsstelle oder deren Umgebung, basierend auf Daten für beide AFLP Systemen;
21) Identifizierung von Ereignissen, besonders der Aktivität der Faktoren induzierenden Variabilität in *in vitro* Kulturen unterworfen;
22) Sequenzanalyse der einzelnen Ereignistypen und/oder der breite Sequenzvariabilität, wo Variabilität wird mit AFLP-Technik Varianten unter Verwendung von Primern gegen konservativen Sequenzen innerhalb beweglichen Elemente gerichtete, sich wiederholenden Sequenzen oder Sequenzen, die konservative Genfamilien identifiziert;
23) statistische Analyse der Donorpflanze und ihre Regeneranten sowie Regeneranten-Nachkommen und möglicherweise unterschiedliche Regeneranten und Regeneranten-Nachkommen zusammen mit den entsprechenden Donorpflanzen sowie AFLP Reproduzierbarkeit Kontrolle und die Kontrolle der generativen Vermehrung:
a) statistische Analyse der Ergebnisse der AFLP unter Verwendung der parametrischen Analyse, Mantel-Test und/oder Cronbachs Alpha-Koeffizient sowie molekularen Varianz-Analyse mit dem Fs oder Phis Koeffizienten;
b) Bestimmung des Fehlers der induzierte Gewebekultur und somaklonale Variabilität Schätzungen, basierend auf der Mantel-Test Korrelationskoeffizient;
c) Bestimmung der quantitativen Parametern der Variabilität induziert durch das Verfahren der Ableitung von Regeneranten und somaklonale Variabilität von Regeneranten, von einzelnen Regeneranten-Nachkommen, unter Berücksichtigung der Methode der Ableitung und gebend ihre statistische Signifikanz Ebene;
d) Statistik Bestimmung der Unterschiede zwischen den einzelnen Ereignistypen induziert *in in* vitro-Kulturen für Regeneranten, sowie Massen-Regeneranten Nachkommen, durch die Methode der Ableitung gruppierten und ergänzt durch ihre statistische Signifikanz anhand quantitativer Matrizen von Ereignissen;
e) statistische Analyse des Vergleichs zwischen Variabilität induziert durch das Verfahren der Ableitung sowie somaklonale Variation bestimmte durch das Vergleich des Profils der Donorpflanze, und des Profils der Masse von Regeneranten-Nachkommen und deren Typen: Sequenzvariabilität und/oder breite Sequenzvariabilität, *de novo* Methylierung, Demethylierung, gemischte Variabilität, Vererbung von: Methylierungsmuster, nicht-methylierte Stellen oder Stellen in konservativen Regeneranten-Nachkommen, gruppiert durch die Methode der Ableitung sowie einem Vergleich mit der Methode der Ableitung der Regeneranten-Nachkommen, wo die quantitative Matrizen für Ereignissen genutzt werden;
f) statistische Vergleiche von Regeneranten und die Masse von Regeneranten-Nachkommen für verschiedene Methoden der Ableitung für einen bestimmten Regenerante-Nachkommen, wobei quantitative Matrizen der Nachkommen verwendet werden;
g) statistisches Vergleich der Ergebnisse der oben genannten Punkte für einzelnen Regeneranten und Massen-Regeneranten Nachkommen untereinander;
h) statistischer oder qualitativer Vergleich der somaklonalen Variation zwischen einzelnen Pflanzen als generative Nachkommen von Regeneranten;
i) Zusammenführen der vorliegenden Ergebnisse in Form einer Zusammenstellung anzeigt quantitativen Eigenschaften von der Variabilität durch das Verfahren der Ableitung am Regeneranten Ebene sowie quantitative Eigenschaften von geerbter Variabilität auf der Ebene von Regeneranten-Nachkommen bestimmt induziert.

2. Verwendung nach Anspruch 1, wobei die Identifizierung der 14 Ereignistypen auf der Ebene der binären Matrix erkannt, wird aus den Ereignissen in der Fig. 7 dargestellt ausgewählt.

3. Verwendung nach Anspruch 1, wobei zum Ableiten der Donorpflanzen wird eine einzelne Pflanze verwendet, der doppelte Haploiden aus einer isolierten Mikrosporen abgeleitet, wobei es als Kontrolle der genetischen und epigenetischen Variabilität der Donorpflanzen behandelt ist.

4. Verwendung nach Anspruch 1, wobei genetische und epigenetische Variabilität von generativ vermehrtem Material, Donorpflanzen, basierend auf AFLP Profile erhaltene von Restriktionsendonuklease Isoschizomere, die identischen Sequenzen erkennen und verdauen, aber die sich in ihrer Empfindlichkeit gegenüber Methylierung an oder über der Restriktionsstelle unterscheiden bestimmt wird, wobei eine der Isoschizomere unempfindlich gegenüber Methylierung ist.

5. Verwendung nach Anspruch 1, wobei in einem empfindlichen und unempfindlichen gegen Methylierung System keine Unterschiede in den AFLP Profile der Donorpflanzen beobachtet sind, ist dann die Variabilität Niveau induziert durch generative Vermehrung und identifiziert mit einem gegebenen Paar der selektiven Primer unerheblich.

6. Verwendung nach Anspruch 1, wobei, wenn es die Unterschiede in AFLP Profile für Donorpflanzen zwischen einem empfindlichen und unempfindlichen gegen Methylierung System gibt, wird eine prozentuale Anteil eines solchen Ereignisses bestimmt wird, zusammen mit dem Hintergrundniveau, generativem Vermehrungsmaterial Fehler.

7. Verwendung nach Anspruch 1, wobei, wenn es die Unterschiede in AFLP Profile für experimentelle Replikanten auf dem gleichen DNA-Proben gibt, die AFLP-Technik Fehler geschätzt wird, um experimentelle Fehler zu bestimmen.

8. Verwendung nach Anspruch 1, wobei das Integral des Forschungsmaterial aus der Donorpflanzen, zusammen mit einer einzigen Pflanze, Donorpflanze Regeneranten und separater Regeneranten-Nachkommen besteht.

9. Verwendung nach Anspruch 1, wobei die Donorpflanze mit Regeneranten und Regeneranten-Nachkommen bilden den Kern der Forschung Material für die Untersuchung der Variabilität durch das Verfahren der Ableitung oder somaklonale Variabilität induzierte.

10. Verwendung nach Anspruch 1, wobei die molekulare Analyse unter Verwendung der AFLP-Technik in zwei Varianten durchgeführt wird, unter Verwendung der Isoschizomeren, die sich in ihrer Empfindlichkeit gegenüber Methylierung unterscheiden.

11. Verwendung nach Anspruch 1, wobei einer der Isoschizomere völlig unempfindlich gegen Methylierung ist.

12. Verwendung nach Anspruch 1, wobei bei der Identifizierung von getrennten PCR-Produkten, und Isotopen-Markierung verwendet wird, die an das 5'-Ende des Primers komplementär zu dem Adapter der befreiten durch die Isoschizomere Restriktionsstelle geglüht eingeführt ist

13. Verwendung nach Anspruch 1, wobei andere Methoden der Visualisierung AFLP-Fragmente verwendet werden, einschließlich der Fluoreszenz.

14. Verwendung nach Anspruch 1, wobei die Profile der beiden AFLP Varianten für die Donorpflanze zusammen mit der Regeneranten und unter Berücksichtigung des Verfahrens der Ableitung und AFLP Reproduzierbarkeit Kontrolle mit gleichen AFLP-Primer-Paaren durchgeführt werden.

15. Verwendung nach Anspruch I, bei AFLP-Profil-Analyse und ihre Umwandlung in binäre Matrizen auf der Tatsache basiert, dass die AFLP Profile beider Restriktasesystemen für alle Paare von selektiven Primer als binäre Matrix in eine Tabellenkalkulation, wo die Daten erhaltenen für ein Paar von selektiven AFLP-Primern unter dem vorherigen gesetzt werden, so dass jede Spalte insgesamt binären Profil von einer Donorpflanze in einem ALFP-System darstellt, und die nachfolgenden Spalten analoge Daten für Regeneranten und Kontrollen enthalten, wobei die Daten für den zweiten AFLP-System in den folgenden Spalten dargestellt werden, wobei die Bänder von einem System sind wie die aus dem bisherigen System (in Reihen) angeordnet, und wenn eine bestimmte Band nicht erscheint, wird eine Null in die entsprechende Zeile der Tabelle eingetragen.

16. Verwendung nach Anspruch I, bei AFLP-Profil-Analyse und ihre Umwandlung in binäre Matrizen auf der Tatsache basiert, dass die AFLP Profile beider Restriktasesystemen für alle Paare von selektiven Primer als binäre Matrix in eine Tabellenkalkulation, wo die Daten erhaltenen für ein Paar von selektiven AFLP-Primern unter dem vorherigen gesetzt werden, so dass jede Spalte insgesamt binären Profil von einer Donorpflanze in einem ALFP-System darstellt, und die nachfolgenden Spalten analoge Daten für Regeneranten-Nachkommen und Kontrollen enthalten, wobei die Daten für den zweiten AFLP-System in den folgenden Spalten dargestellt werden, wobei die Bänder von einem System sind wie die aus dem bisherigen System (in Reihen) angeordnet, und wenn eine bestimmte Band nicht erscheint, wird eine Null in die entsprechende Zeile der Tabelle eingetragen.

17. Verwendung nach Anspruch 1, wobei das AFLP-Profil von Regeneranten-Nachkommen wird in Form einer Bulk Genotyp pro AFLP Variante empfindlich und unempfindlich gegen Methylierung getrennt dargestellt.

18. Verwendung nach Anspruch 1, wobei die Kontrolle fur die generative Vermehrung wird durch die Summe der Anzahl der Signale (Ereignisse) von einer einzigen Pflanze durchgeführt, wenn die Anzahl der Änderungen in Prozent der Gesamtzahl der Ereignisse berechnet wird.

19. Verwendung nach Anspruch 1, wobei die parametrische Analyse Mantel-Test Korrelationsanalyse und molekulare Variabilität Analyse unter Verwendung von Binärmatrizen von AFLP Systemen empfindlich und unempfindlich gegen Methylierung für Regeneranten und/oder Regeneranten-Nachkommen durchgeführt sind, je nachdem, ob die Variabilität induziert in Kultur oder vererbt von Regeneranten-Nachkommen untersucht wird.

20. Verwendung nach Anspruch 1, wobei die faktorielle Analyse als graphische Darstellung der AFLP Wirkung verwendet ist.

21. Verwendung nach Anspruch 1, wobei die Identifizierung der 14 Ereignistypen auf der Ebene der AFLP-Muster erkannt und der Variabilität zugeordnet, die durch das Verfahren der Ableitung induziert ist, wird auf der Grundlage der AFLP-Mustern durchgeführt, wobei die Donorpflanze Profil zusammen mit Regeneranten-Profile in beiden Systemen verwendet wird, und anschließend das Profil jedes Regeneranten dem Donorpflanze Profil verglichen werden, um diese Ereignisse zu identifizieren.

22. Verwendung nach Anspruch 1, wobei die Identifizierung der 14 Arten von Ereignissen auf der Ebene der AFLP Mustern erkannt und der somaklonalen Variabilität zugeordnet, wird auf der Grundlage der AFLPMustern durchgeführt, wobei der Donorpflanzeprofil verwendet wird mit Profilen von beiden AFLP-Systemen und die Masse Profil Regeneranten-Nachkommen mit dem Donorpflanze Profil verglichen, um diese Ereignistypen zu identifizieren, wobei angenommen wird, dass im Falle einer polymorphen AFLP-Fragmente des Massen-Regeneranten Nachkommen Genotyp, ein anderer Wert als der des Donorpflanze eingegeben wird oder ein anderer Wert, wenn es sich von der der Donorpflanze in allen Regeneranten-Nachkommen für eine gegebene AFLP-System ist.

23. Verwendung nach Anspruch 1, wobei, wenn die 14 Ereignistypen die Grundlage für ihre Trennung in Gruppen umfassen: Sequenzvariabilität, Methylierung Variabilität (Methylierung und Demethylierung), gemischte Variabilität und Vererbung von Methylierungsmuster, wobei berücksichtigt wird, dass Sequenzvariabilität unmissverständlich in einem System unempfindlich gegenüber Methylierung bestätigt ist.

24. Verwendung nach Anspruch 1, wobei die Analyse zum Stellenwert der individuellen Variabilität und Ereignissen innerhalb der Regeneranten und Regeneranten-Nachkommenpflanzen, nach dem Verfahren der Ableitung gruppiert sind, basierend auf qualitative und quantitative Matrizen und quantitativen Matrizen von Ereignissen durchgeführt, die alle theoretisch möglichen Ereignissen, sowie Regeneranten-Pflanzen und Regeneranten-Nachkommen beschreiben.

25. Verwendung nach Anspruch 1, wobei die Ergebnisse der statistischen Analyse für individuelle Regeneranten und Massen-Regeneranten-Nachkommen durch verschiedene Mittel erreichte, sind zu einem Ganzen zusammengefasst; eingegeben sind quantitative Parameter für die Variabilität durch das Verfahren der Ableitung induziert und somaklonaler Variabilität für individuelle Regeneranten und Massen-Regeneranten-Nachkommen sowie für getrennte Regeneranten Nachkommen nach der Methode der Ableitung und Ereignistypen zusammen mit statistischen Parameter gruppiert.

26. Verwendung nach Anspruch 1, wobei diese Ereignistypen identifiziert sind, die selten in Kultur oder die überhaupt nicht auftreten, als auch solche, die besonders anfällig für die Aktivität der Faktoren induzierenden Variabilität auf der Ebene der Regeneranten und Regeneranten-Nachkommen sind, sowie ihre quantitative Parameter zu bestimmen.

27. Verwendung nach Anspruch 1, wobei einzelne Ereignistypen auf der molekularen Ebene durch die Sequenzanalyse von AFLP-Fragmente untersucht werden, die einzelne Ereignistypen, und aus dem gleichen Grund Arten von somaklonale Variabilität representieren.

28. Verwendung nach Anspruch 1, wobei ein oder mehrere Massen-Regeneranten-Nachkommen analysiert werden.

29. Verwendung nach Anspruch 1, wobei die quantitativen Eigenschaften eines Ereignisses für jeden einzelnen Regeneranten-Nachkommen angegeben sind: Sequenzvariabilität, breite Sequenzvariabilität, epigenetische Variabilität der Methylierungsmuster *(de novo* Methylierung, Demethylierung), Stabilität der vererbten methylierter und nicht-methylierter Stellen (konservativ), gegen Änderungen in Kulturen sowie die Eingabe der quantitativen Unterschiede in Variabilität zwischen verschiedenen Methoden der Ableitung des Pflanzenmaterials induzierten.

30. Verwendung nach Anspruch 1, wobei die statistische Analyse der Bedeutung der einzelnen Ereignistypen unter den Regeneranten, Massen- Regeneranten-Nachkommen und individuelle Regeneranten Nachkommen durchgeführt wird, wie es benötigt wird, gruppierten nach der Methode der Ableitung der Forschung Material.

31. Verwendung nach Anspruch 1, wobei die somaklonaler Variabilität Ebene der einzelnen Pflanzen, die Regeneranten-Nachkommen wird unabhängig bestimmt, unter Verwendung der Prinzipien für somaklonale Variabilität verwendet.

32. Verwendung nach Anspruch 1, wobei die Hypothesen zu den Quellen der Variabilität durch das Verfahren der Ableitung Regeneranten induziert und die vererbte als Ergebnis der generatives Vermehrungsmaterial getestet.

33. Verwendung nach Anspruch 1, wobei die detaillierte Analyse der Ursachen von Sequenzvariabilität nach Einführung kleinere AFLP Verfahrensänderungen durchgeführt wird, basierend auf der selektiven Amplifikation des Forschungsmaterials unter Verwendung eines markierten Primers, die komplementär zu konservativen Seiten von Transposons oder Macrosatellitesequenzen sind.

34. Verwendung nach Anspruch 1, wobei die Trennung von genetischen und epigenetischen Marker für Mappingpopulationen durch Pflanz-Pflanz Kreuze abgeleitet ist, welche die Nachkommen von Regeneranten mit einem bekannten Genotyp abgeleitet werden, basierend auf der AFLP Profile für beide AFLP Systemen.

35. Verwendung nach Anspruch 1, wobei das Verfahren zum Ableiten von Pflanzen *in vitro* mit einem abgesenkten oder gestiegenen Niveau der somaklonalen Variabilität durch die Identifizierung der Ursachen dieser Variabilität entwickelt ist.

36. Verwendung nach Anspruch 1, wobei die genetische und epigenetische Reinheit von beliebigem Material *durch in vitro* Kultur abgeleitete untersucht wird.

37. Verwendung nach Anspruch 1, wobei, wenn das Verfahren in der Analyse der Vererbung von epigenetischen Variabilität während der sexuellen Kreuzung verwendet wird.

38. Verwendung nach Anspruch 1, wobei das Verfahren ein Werkzeug zur Identifizierung von epigenetischen Markierungen verknüpft mit den nützliche Eigenschaften des pflanzlichen Materials ist.

39. Verwendung nach Anspruch 1, wobei das Verfahren zur Identifizierung von Unterschieden in der DNA-Methylierungsmuster zu verschiedenen Geweben einer einzelnen Pflanze verwendet wird.

## Revendications

1. Utilisation de la technique AFLP qui se compose des étapes: l'isolement de l'ADN génomique, la digestion de l'ADN génomique par une paire d'endonucléases de restriction et la digestion d'une autre partie du même ADN par une autre paire d'endonucléases de restriction, l'assemblage de heteroduplexes synthétiques aux extrémités de restriction libres, l'amplification initiale des fragments obtenus de telle manière au moyen de la PCR primaire, l'amplification sélective, la séparation des produits de PCR dans un gel de polyacrylamide, l'analyse visuelle du profil pour le procédé pour l'identification de la variabilité induite par le procédé de dérivation dans les *cultures in vitro* au niveau des plantes régénérantes, ainsi que la variation héritée à la suite de la propagation générative comprenant les étapes suivantes:
1) estimation de la répétabilité de ces deux variantes de la technique AFLP: contrôle de la reproductibilité des AFLP sur la base de l'identité de plusieurs échantillons d'ADN de la même plante par l'utilisation d'AFLP et des isoschizomères à la fois sensibles et insensibles à la méthylation au niveau du site de restriction et/ou les zones adjacentes ainsi qu'un nombre représentatif de paires d'amorces sélectives identiques dans toutes les analyses AFLP;
2) estimation de la répétabilité de ces deux variantes de la technique AFLP: contrôle de la reproductibilité des AFLP sur la base de l'identité de plusieurs échantillons d'ADN de la même plante par l'utilisation d'AFLP et des isoschizomères à la fois sensibles et insensibles à la méthylation au niveau du site de restriction et/ou les zones adjacentes ainsi qu'un nombre représentatif de paires d'amorces sélectives en utilisant un ensemble différent d'amorces AFLP sélectifs que ceux utilisés dans les autres analyses AFLP;
3) identification de la variabilité induite par le procédé de dérivation des régénérants par rapport à la plante donneuse est réalisée en deux variantes AFLP en utilisant des isoschizomères à la fois sensibles et insensibles à la méthylation au niveau du site de restriction et/ou les zones adjacentes ainsi qu'un nombre représentatif de paires d'amorces sélectives identiques dans toutes les analyses AFLP;
4) l'identification de la variabilité induite par la culture de tissus parmi les régénérants par rapport à la plante donneuse est réalisée en deux variantes de la technique AFLP en utilisant des isoschizomères à la fois sensibles et insensibles à la méthylation au niveau du site de restriction et/ou les zones adjacentes ainsi qu'un nombre représentatif de paires d'amorces sélectives identiques dans toutes les analyses AFLP;
5) l'identification de la variabilité somaclonale parmi la progéniture de régénérant par rapport à la plante donneuse est réalisée en utilisant deux variantes de la technique AFLP en utilisant des isoschizomères à la fois sensibles et insensibles à la méthylation au niveau du site de restriction et/ou les zones adjacentes ainsi qu'un nombre représentatif de paires d'amorces sélectives identiques dans toutes les analyses AFLP;
6) l'analyse moléculaire du matériel expérimental en utilisant la technique AFLP en tenant compte des isoschizomères à la fois sensibles et insensibles à la méthylation au site de restriction et/ou les zones adjacentes ainsi qu'un nombre représentatif de paires d'amorces sélectives identiques dans toutes les analyses AFLP est constituée des étapes suivantes:
a) analyses des modèles AFLP de plantes donneuses par rapport à la variabilité génétique et épigénétique induite lors de la propagation générative d'une plante individuelle, un contrôle de la propagation générative,
b) analyses des modèles AFLP pour le contrôle de la reproductibilité de ces deux variantes de la technique AFLP,
c) analyse des modèles AFLP pour les régénérants par comparaison aux plantes donneuses ou les régénérants distincts par rapport aux plantes donneuses appropriées;
d) analyses des modèles AFLP pour une plante donneuse, son régénérant et la progéniture de régénérant, ou des lignes distinctes de progéniture de régénérant ainsi qu'une donatrice appropriée et ses plantes régénérantes;
7) identification des 14 types d'événements reconnus au niveau de la matrice binaire pour des systèmes sensibles et insensibles à la méthylation, qui sont le résultat d'une comparaison entre la plante donneuse avec les régénérants et entre la plante donneuse avec la progéniture de régénérant simultanément dans deux systèmes AFLP, dans laquelle l'identification des types d'événement est réalisée individuellement pour chaque régénérant et volumiquement pour les progénitures de régénérant et est représentée comme 14 matrices binaires qui décrivent chacun de ces événements de façon indépendante, subdivisées en procédés de dérivation; dans lesquelles les colonnes représentent les régénérants consécutifs ou les volumes et les lignes représentent les fragments AFLP consécutifs;
8) attribution de certains types d'événement à un type donné de la variabilité induite par le procédé de dérivation par rapport à chacun régénérant;
9) identification des 14 types d'événements reconnus au niveau de la matrice binaire pour des systèmes sensibles et insensibles à la méthylation, qui sont le résultat d'une comparaison entre la plante donneuse avec le régénérant simultanément dans deux systèmes AFLP;
10) identification des 14 types d'événements reconnus au niveau de la matrice binaire pour des systèmes sensibles et insensibles à la méthylation, qui sont le résultat d'une comparaison entre la plante donneuse avec la progéniture de régénérant simultanément dans deux systèmes AFLP, où l'identification des types d'événement est réalisée par la sommation des génotypes d'une progéniture sommaire donnée, et par la comparaison d'un tel profil AFLP total de la progéniture de régénérant avec le profil de la plante donneuse,
11) le génotype volumique de la progéniture de régénérant exprimé par l'intermédiaire d'un profil AFLP pour des systèmes sensibles et insensibles à la méthylation au niveau des fragments individuels d'AFLP a une valeur différente de celle de la plante donneuse dans un système d'AFLP donné, pour autant qu'un fragment donné en son sein est polymorphe, ou est totalement différent de cette valeur pour la plante donneuse; dans le cas contraire les valeurs pour la plante donneuse et la progéniture volumique de régénérant pour un fragment donné d'AFLP dans un système donné d'AFLP sont identiques,
12)production d'une matrice quantitative pour les régénérants, où les colonnes contiennent des données pour les régénérants consécutifs regroupés par le procédé de dérivation, et les lignes contiennent les 14 types d'événements qui peuvent être attribués à chaque progéniture de régénérant;
13)production d'une matrice quantitative pour la progéniture de régénérant, où les colonnes contiennent des données volumiques pour la progéniture de régénérant regroupée par le procédé de dérivation, et les lignes contiennent les 14 types d'événements qui peuvent être attribués à chaque progéniture volumique de régénérant;
14)production d'une matrice quantitative pour les événements dans les régénérants, où il s'agit d'une matrice quantitative pour les régénérants après transposition, où les colonnes contiennent 14 événements et les lignes contiennent les régénérants regroupés par le procédé de dérivation;
15)calcul du nombre total d'événements sur la base de la matrice binaire pour des procédés individuels de dérivation des régénérants ainsi que pour les régénérants, où le nombre total d'événements est calculé comme un produit du nombre de fragments d'AFLP identifiés dans les deux systèmes d'AFLP pour toutes les paires d'amorces sélectives multiplié par le nombre de régénérants dérivé par un procédé donné;
16)calcul du nombre total d'événements sur la base de la matrice binaire pour des procédés individuels de dérivation des régénérants ainsi que pour les progénitures volumiques de régénérant, où le nombre total d'événements correspond au nombre de fragments d'ADN, identiques ou différents, dans les deux systèmes d'AFLP pour toutes les paires d'amorces sélectives utilisées dans l'analyse;
17)calcul de l'erreur résultant de la propagation générative est réalisé sur la base de la matrice binaire de plantes donneuses et une plante individuelle; détermination du nombre d'événements différents de ceux d'une plante individuelle appartenant aux plantes donneuses, et le calcul du nombre total d'événements pour les plantes donneuses;
18)calcul de l'erreur résultant de la technique AFLP est réalisé sur la base de la matrice binaire pour les échantillons d'ADN obtenus à partir de la même plante et soumis aux étapes de la technique AFLP en utilisant des isoschizomères sensibles et insensibles à la méthylation au site de restriction ou ses environs immédiats ainsi qu'un nombre représentatif de paires d'amorces sélectives identiques dans tous les systèmes AFLP, où le nombre total d'événements et le nombre d'événements polymorphes sont calculés, et le second nombre est exprimé sous forme de pourcentage;
19)détermination, basée sur des matrices binaires pour les régénérants et la plante donneuse, des groupes d'événements décrivant: (a) variation de séquence liée à toute modification dans les séquences de nucléotides de l'ADN à partir des données AFLP du système insensible à la méthylation et (b) variabilité épigénétique, la variabilité *de novo* des modèles de méthylation, et en outre la variation mixte qui est la variabilité de séquence et de méthylation basée sur des données pour les deux systèmes AFLP;
20) détermination, basée sur des matrices binaires pour la progéniture volumiques de régénérant et la plante donneuse, de groupes d'événements décrivant (a) variation de séquence liée à toute modification dans les nucléotides de l'ADN à partir des données du système insensible à la méthylation et (b) variabilité épigénétique, la variabilité *de novo* des modèles de méthylation, et en outre la variation mixte qui est la variabilité de séquence et de méthylation, et (c) héritage de modèles de méthylation comme lié à la présence et/ou l'absence de méthylation au niveau du site de restriction ou de son voisinage, basé sur des données pour les deux systèmes AFLP;
21) identification d'événements particulièrement soumises à l'activité des facteurs induisant la variabilité dans les *cultures in vitro;*
22) analyse de séquence de types d'événements individuels et/ou grande variabilité de séquence, où la variabilité est identifié en utilisant des variantes de la technique AFLP en utilisant des amorces dirigées contre des séquences conservatrices au sein des éléments mobiles, séquences répétitives ou les séquences de familles de gènes conservateurs;
23) analyse statistique de la plante donneuse et ses régénérants ainsi que la progéniture de régénérant, et éventuellement les régénérants distincts et les progénitures de régénérant ainsi que les plantes donneuses appropriées, aussi bien que le contrôle de la reproductibilité AFLP et le contrôle de la propagation générative:
a) analyse statistique des résultats des AFLP en utilisant une analyse paramétrique, le test de Mantel et/ou le coefficient alpha de Cronbach, ainsi que l'analyse de la variance moléculaire en utilisant les coefficients *Fₛ*, ou *Phiₛ;*
b) détermination de l'erreur de la culture de tissus induite et des estimations de la variabilité somaclonale, sur la base du coefficient de corrélation du test de Mantel;
c) détermination des paramètres quantitatifs de la variabilité induite par le procédé de dérivation des régénérants et la variabilité somaclonale pour les régénérants, les progénitures individuelles de régénérant, en tenant compte du procédé de dérivation et en donnant leur niveau de signification statistique;
d) détermination statistique des différences entre les types particuliers d'événements induits dans les *cultures in vitro* pour les régénérants ainsi que la progéniture volumique de régénérant, regroupés par le procédé de dérivation et complétés par leur signification statistique en utilisant des matrices quantitatives des événements;
e) analyse statistique de la comparaison entre la variabilité induite par le procédé de dérivation ainsi que la variation somaclonale, déterminée par la comparaison du profil de la plante donneuse, et le profil volumique de la progéniture de régénérant et de leurs types: variabilité des séquences et/ou grande variabilité de séquence, méthylation de *novo,* déméthylation, variabilité mixte, héritage de: modèles de méthylation, sites non méthylés ou sites conservateurs dans la progéniture de régénérant, regroupés par le procédé de dérivation ainsi qu'une comparaison par le procédé de dérivation de la progéniture de régénérant, où les matrices quantitatives sont utilisées pour des événements;
f) comparaisons statistiques des régénérants et les progénitures volumiques de régénérant pour les différentes procédé de dérivation pour une progéniture donnée de régénérant, où les matrices quantitatives de la progéniture sont utilisées;
g) comparaison statistique des résultats des points ci-dessus mentionnés pour régénérants individuels et progénitures volumiques de régénérant entre eux;
h) comparaison statistique ou qualitative de la variation somaclonale entre les plantes individuelles étant la progéniture générative de régénérants;
i) classement des résultats existants sous forme d'une compilation indiquant des caractéristiques quantitatives de la variabilité induite par le procédé de dérivation déterminées au niveau de régénérants, ainsi que des caractéristiques quantitatives de la variabilité héritée déterminées au niveau de la progéniture de régénérant.

2. Utilisation selon la revendication 1, dans laquelle l'identification des 14 types d'événements reconnus au niveau de la matrice binaire est choisie parmi les événements représentés dans la Fig. 7.

3. Utilisation selon la revendication 1, dans laquelle pour dériver plantes donneuses, une seule plante est utilisée qui est un haploïde doublé dérivé d'une microspore isolée, dans laquelle elle est traitée comme un contrôle de la variabilité génétique et épigénétique des plantes donneuses.

4. Utilisation selon la revendication 1, dans laquelle la variabilité génétique et épigénétique du matériel propagé de façon générative, plantes donneuses, est déterminée sur la base de profils AFLP obtenus à partir des isoschizomères d'endonucléase de restriction reconnaissant et digérant des séquences identiques, mais différant dans leur sensibilité à la méthylation à ou à environ du site de restriction, dans laquelle l'un des isoschizomères est insensible à la méthylation.

5. Utilisation selon la revendication 1, dans laquelle dans un système sensible et insensible à la méthylation aucune différence n'est observée dans les profils AFLP des plantes donneuses, alors le niveau de variabilité induite par la propagation générative et identifié par une paire donnée d'amorces sélectives est insignifiant.

6. Utilisation selon la revendication 1, dans laquelle quand il y a des différences dans les profils AFLP pour plantes donneuses entre un système sensible et un système insensible à la méthylation, une partie en pourcentage d'un tel événement est déterminée, ainsi que le niveau de fond, erreur de propagation générative.

7. Utilisation selon la revendication 1, dans laquelle quand il y a des différences dans les profils AFLP pour réplicants expérimentaux sur les mêmes échantillons d'ADN, l'erreur de la technique AFLP est estimée afin de déterminer l'erreur expérimentale.

8. Utilisation selon la revendication 1, dans laquelle la partie intégrale du matériel de recherche est composée des plantes donneuses, ainsi qu'une seule plante, des régénérants de plantes donneuses et des progénitures distinctes de régénérant.

9. Utilisation selon la revendication 1, dans laquelle la plante donneuse ainsi que les régénérants et la progéniture de régénérant composent le noyau du matériel de recherche pour l'étude de la variabilité induite par le procédé de dérivation ou de la variabilité somaclonale, respectivement.

10. Utilisation selon la revendication 1, dans laquelle l'analyse moléculaire est réalisée en utilisant la technique AFLP en deux variantes, en utilisant les isoschizomères se différenciant par leur sensibilité à la méthylation.

11. Utilisation selon la revendication 1, dans laquelle l'un des isoschizomères est totalement insensible à la méthylation.

12. Utilisation selon la revendication 1, dans laquelle dans l'identification des produits séparés de PCR, et traceur isotopique est utilisé, qui est introduit à l'extrémité 5 'de l'amorce complémentaire à l'adaptateur joint au site de restriction libéré par les isoschizomères.

13. Utilisation selon la revendication 1, dans laquelle d'autres procédés de visualisation des fragments AFLP sont utilisés, notamment la fluorescence.

14. Utilisation selon la revendication 1, dans laquelle les profils des deux variantes AFLP pour la plante donneuse ainsi que ses régénérants et en tenant compte du procédé de dérivation et le contrôle de la reproductibilité AFLP sont réalisés sur les paires identiques d'amorces AFLP.

15. Utilisation selon la revendication 1, dans laquelle l'analyse des profils AFLP et leur conversion en matrices binaires est basée sur le fait que les profils AFLP des deux systèmes de restrictase pour toutes les paires d'amorces sélectives sont entrés en tant que matrice binaire dans un tableur, où l'ensemble des données obtenues pour une paire d'amorces sélectives AFLP est placé sous un ensemble précédent de telle sorte que chaque colonne représente un profil binaire total pour une plante donneuse dans un système AFLP, et les colonnes suivantes contiennent des données analogues pour les régénérants et les contrôles, dans laquelle les données pour le deuxième système AFLP sont enregistrées dans les colonnes suivantes, dans laquelle les bandes d'un système sont ordonnées comme celles du système précédent (en lignes), et où une bande donnée n'apparaît pas un zéro est entré dans la ligne correspondante du tableur.

16. Utilisation selon la revendication 1, dans laquelle l'analyse des profils AFLP et leur conversion en matrices binaires est basée sur le fait que les profils AFLP des deux systèmes de restrictase pour toutes les paires d'amorces sélectives sont entrés en tant que matrice binaire dans un tableur, où l'ensemble des données obtenues pour une paire d'amorces sélectives AFLP est placé sous un ensemble précédent de telle sorte que chaque colonne représente un profil binaire total pour une plante donneuse dans un système AFLP, et les colonnes suivantes contiennent des données analogues pour les progénitures de régénérant et les contrôles, dans laquelle les données pour le deuxième système AFLP sont enregistrées dans les colonnes suivantes, dans laquelle les bandes d'un système sont ordonnées comme celles du système précédent (en lignes), et où une bande donnée n'apparaît pas un zéro est entré dans la ligne correspondante du tableur.

17. Utilisation selon la revendication 1, dans laquelle le profil AFLP pour la progéniture de régénérant est représenté sous forme d'un génotype volumique par variante AFLP, sensible et insensible à la méthylation séparément.

18. Utilisation selon la revendication 1, dans laquelle le contrôle de la propagation générative est réalisé par la sommation du nombre de signaux (événements) d'une seule plante, où le nombre de modifications est calculé comme un pourcentage du nombre total d'événements.

19. Utilisation selon la revendication 1, dans laquelle l'analyse paramétrique, l'analyse de corrélation de test de Mantel et l'analyse de la variabilité moléculaire sont réalisées en utilisant des matrices binaires de systèmes AFLP sensibles et insensibles à la méthylation pour les régénérants et/ou la progéniture de régénérant selon que la variabilité induite dans la culture ou héritée par la progéniture de régénérant sont en cours d'analyse.

20. Utilisation selon la revendication 1, dans laquelle l'analyse factorielle est utilisée en tant que la représentation graphique des résultats de l'AFLP.

21. Utilisation selon la revendication 1, dans laquelle l'identification des 14 types d'événements reconnus au niveau du modèle AFLP et attribués à la variabilité induite par le procédé de dérivation est effectuée sur la base des modèles AFLP, dans laquelle le profil de la plante donneuse est utilisé avec des profils des régénérants dans les deux systèmes, et ensuite le profil de chaque régénérant est comparé au profil de la plante donneuse afin d'identifier ces événements.

22. Utilisation selon la revendication 1, dans laquelle l'identification des 14 types d'événements reconnus au niveau des modèles AFLP et attribués à la variabilité somaclonale est effectuée sur la base des modèles AFLP, dans laquelle le profil de la plante donneuse est utilisé avec des profils dans les deux systèmes AFLP, et le profil volumique de la progéniture de régénérant est comparé au profil de la plante donneuse afin d'identifier les types d'événements, dans laquelle on suppose que dans le cas de fragments polymorphes d'AFLP du génotype volumique de la progéniture de régénérant, une valeur différente de celle de la plante donneuse est entrée ou une autre valeur si elle est différente de celle de la plante donneuse dans toutes les progénitures de régénérant pour un système donnée d'AFLP.

23. Utilisation selon la revendication 1, dans laquelle lorsque les 14 types d'événements comprennent la base pour leur ségrégation dans les groupes: variabilité de séquence, variabilité de méthylation (méthylation et déméthylation), variabilité mixte et héritage des modèles de méthylation, dans laquelle il est pris en compte le fait que la variabilité de séquence est confirmée sans équivoque dans un système insensible à la méthylation.

24. Utilisation selon la revendication 1, dans laquelle l'analyse de la signification des types individuels de variabilité et des événements au sein des régénérants et des plantes de la progéniture de régénérant, comme regroupés par le procédé de dérivation, est effectuée sur la base des matrices qualitatives et des matrices quantitatives d'événements décrivant tous les événements théoriquement possibles, ainsi que les plantes de régénérant et la progéniture de régénérant.

25. Utilisation selon la revendication 1, dans laquelle les résultats de l'analyse statistique pour les régénérants individuels et les progénitures volumiques de régénérant obtenus par divers moyens sont compilés dans un ensemble; paramètres quantitatifs sont entrés pour la variabilité induite par le procédé de dérivation et de la variabilité somaclonale pour les régénérants individuels et les progénitures volumiques de régénérant, ainsi que pour les progénitures distinctes de régénérant regroupées par le procédé de dérivation et les types d'événements avec paramètres statistiques.

26. Utilisation selon la revendication 1, dans laquelle ces types d'événements sont identifiés, qui sont rares dans la culture ou qui ne se produisent pas du tout, ainsi que ceux qui sont particulièrement exposés à l'activité des facteurs induisant la variabilité au niveau du régénérant et la progéniture de régénérant, ainsi que pour déterminer leur paramètres quantitatifs.

27. Utilisation selon la revendication 1, dans laquelle types individuels d'événements sont étudiés au niveau moléculaire par l'analyse de séquence des fragments AFLP représentant types individuels d'événements, et par les mêmes types de marqueurs de la variabilité somaclonale.

28. Utilisation selon la revendication 1, dans laquelle on analyse une ou plus de progénitures volumiques de régénérant.

29. Utilisation selon la revendication 1, dans laquelle les caractéristiques quantitatives d'un événement sont données pour chaque progéniture individuelle de régénérant: variabilité de séquence, grande variabilité de séquence, variabilité épigénétique des modèles de méthylation (méthylation *de novo,* déméthylation), stabilité des sites hérités méthylés et non méthylés (conservateurs), contre les modifications induites dans les cultures, ainsi que des différences quantitatives entrant dans la variabilité entre les différents procédés de dérivation du matériel de la plante.

30. Utilisation selon la revendication 1, dans laquelle on réalise l'analyse statistique de l'importance des types individuels d'événements parmi les régénérants, la progéniture volumique de régénérant et les progénitures individuels de régénérant si nécessaire, regroupés par le procédé de dérivation du matériel de recherche.

31. Utilisation selon la revendication 1, dans laquelle le niveau de la variabilité somaclonale des plantes individuelles qui sont la progéniture de régénérant est déterminé indépendamment, en utilisant les principes utilisés pour la variabilité somaclonale.

32. Utilisation selon la revendication 1, dans laquelle les hypothèses relatives aux sources de la variabilité induite par le procédé de dérivation des régénérants et celle héritée à la suite de la propagation générative sont testées.

33. Utilisation selon la revendication 1, dans laquelle on réalise l'analyse détaillée des causes de la variabilité de séquence après l'introduction de modifications procédurales mineures d'AFLP, sur la base de l'amplification sélective du matériel de recherche en utilisant une amorce marquée complémentaire aux sites conservateurs des éléments transposables, ou aux séquences macrosatellites.

34. Utilisation selon la revendication 1, dans laquelle la ségrégation de marqueurs génétiques et épigénétiques sont analysés pour mapper les populations dérivées de croisements entre plantes qui sont la progéniture de régénérants avec un génotype connu, sur la base des profils AFLP pour les deux systèmes AFLP.

35. Utilisation selon la revendication 1, dans laquelle le procédé est conçu pour dériver des plantes *in vitro* avec un niveau de la variabilité somaclonale abaissé ou augmenté par l'identification des causes de cette variabilité.

36. Utilisation selon la revendication 1, dans laquelle on vérifie la pureté génétique et épigénétique du matériel arbitraire dérivé par la culture *in vitro.*

37. Utilisation selon la revendication 1, dans laquelle le procédé est utilisé dans l'analyse de l'héritage de la variabilité épigénétique au cours de croisement sexuel.

38. Utilisation selon la revendication 1, dans laquelle le procédé est un outil pour l'identification de marqueurs épigénétiques liés aux caractéristiques utilitaires du matériel de la plante.

39. Utilisation selon la revendication 1, dans laquelle le procédé est utilisé pour l'identification des différences dans les modèles de méthylation de l'ADN pour les différents tissus d'une plante individuelle.
